# EUROPEAN PATENT SPECIFICATION

(11) **EP 1 351 698 B1**
(45) Date of publication and mention of the grant of the patent: **23.01.2013**
(21) Application number: 01994322.4
(22) Date of filing: 18.12.2001
(51) Int. Cl.: A61K 36/537, A61K 36/258, A61P 9/10

(54) **HERBAL COMPOSITION FOR ANGINA PECTORIS, METHOD TO PREPARE SAME AND USES THEREOF**
PFLANZLICHE ZUSAMMENSETZUNG FÜR ANGINA PECTORIS, VERFAHREN ZU IHRER HERSTELLUNG UND IHRE VERWENDUNGEN
COMPOSITION A BASE D'HERBES POUR L'ANGINE DE POITRINE, METHODE DE PREPARATION AFFERENTE ET UTILISATIONS ASSOCIEES

(30) Priority: 22.12.2000 US 258057 P
(43) Date of publication of application: 15.10.2003
(73) Proprietor: Tasly Pharmaceutical Group Co., Ltd., Tianjin 300402 (CN)
(72) Inventor: YAN, Xijun, Hebei District, Tianjin (CN); WU, Naifeng, Hebei District, Tianjin (CN); GUO, Zhixin, Beichen District, Tianjin,P.R China (CN); YE, Zhengliang, Hebei District, Tianjin (CN); LIU, Yan, Hexi District, Tianjin (CN)
(74) Representative: Lecca, Patricia S.
(86) International application number: PCT/US2001/049396
(87) International publication number: WO 2002/058625

(56) References cited:
- US-A- 5 401 502
- US-A- 5 589 182
- LI R ET AL: "Quantitative determination of total tanshinone in salvia miltiorrhiza tablet compound" YIYAO GONGYE - PHARMACEUTICAL INDUSTRY, SHANGHAI, CN, vol. 17, no. 11, 1986, pages 513-514, XP002954785 ISSN: 0255-7223
- NI K ET AL: "Determination of active components of salvia miltiorrhiza and notoginseng in compound salvia miltiorrhiza tablets by TLC-densitometry" YAOWU FENXI ZAZHI - CHINESE JOURNAL OF PHARMACEUTICAL ANALYSIS, ZHONGGUO YAOXUEHUI, BEIJING, CN, vol. 9, no. 2, 1989, pages 74-77, XP002954784 ISSN: 0254-1793
- DATABASE MEDLINE [Online] US NATIONAL LIBRARY OF MEDICINE (NLM), BETHESDA, MD, US; March 1990 (1990-03), LIU Y ET AL: "[Evaluation of radix Salviae Miltiorrhizae and its preparation]" XP002262545 Database accession no. NLM2085402 & ZHONGGUO ZHONG YAO ZA ZHI = ZHONGGUO ZHONGYAO ZAZHI = CHINA JOURNAL OF CHINESE MATERIA MEDICA. CHINA MAR 1990, vol. 15, no. 3, March 1990 (1990-03), pages 159-162, 190, ISSN: 1001-5302
- ANONYMOUS: INTERNET ARTICLE, [Online] XP002262543 Retrieved from the Internet: URL:http://www.carbotrading.com/Products/M edicine/JZ016.htm> [retrieved on 2003-11-18]
- ANONYMOUS: INTERNET ARTICLE, [Online] XP002262544 Retrieved from the Internet: URL:http://www.craneherb.com/products/prod uct001513> [retrieved on 2003-11-18]
- NI K. ET AL.: 'Determination of active components of salvia miltiorrhiza and notoginseng in compound salvia miltiorrhiza tablets by TLC-densitometry' YAOWU FENXI ZAZHI vol. 9, no. 2, 1989, pages 74 - 77, XP002954784
- LIU Y. ET AL.: 'Evaluation of radix salvia miltiorrhiza and its preparation' CHINESE MATERIA MEDICA vol. 15, no. 3, March 1990, pages 159 - 162, XP002903034
- LI R. ET AL.: 'Quantitative determination of total tanshinone in salvia miltiorrhiza tablet compound' YIYAO GONGYE vol. 17, no. 11, 1986, pages 513 - 514, XP002954785
- DATABASE WPI Week 199723, Derwent Publications Ltd., London, GB; AN 1997-246038, XP002954783 LI Y.: 'Heart Protection bag for improving e.g. palpitation of cardialgia' & CN 1 100 657 A 29 March 1995
- YIN, H-G: "Comparative Study of Efficacy of DSP and DST in treatment of Unstable Angina Pectoris", GAN SU CHINESE MEDICINE, 2003,
- "Compound Salvia Tablets (Fufang Danshen Pian)" In: "Pharmacopoeia of the People's Republic of China", 1985 pages 437-438,
- "Compound Salvia Tablets (Fufang Danshen Pian)" In: "Pharmacopoeia of the People's Republic of China", 1990 pages 477-478,
- "Compound Salvia Tablets (Fufang Danshen Pian)" In: "Pharmacopoeia of the People's Republic of China", 1995 pages 545-546,
- "Compound Salvia Tablets (Fufang Danshen Pian)" In: "Pharmacopoeia of the People's Republic of China", 2000 pages 518-519,

## Description

### BACKGROUND OF THE INVENTION

Chronic stable angina pectoris is due to transit myocardial ischemia. Aspirin, nitrates, beta-adrenoceptor blocking drugs, and calcium channel blocking drugs used alone or in combination with one another are the commonly used drugs for angina pectoris.

Aspirin as an antithrombotic agent is a symptomatic treatment for chronic stable angina pectoris.

Nitrates are used to treat an anginal episode and can be successfully used in prophylaxis by patients with predictable symptoms. With all nitrates, troublesome headache can prevent their use in certain susceptible individuals.

Beta-adrenoceptor blocking drugs, although established as a cornerstone in the treatment of angina, are specifically contraindicated in patients with obstructive airways diseases and severe ventricular dysfunction, and relatively contraindicated in diabetes and in those with peripheral vascular disease, bradycardia or heart block.

Calcium antagonists are certainly effective in angina, achieving their effects by smooth muscle relaxation in the coronary arteries and peripheral circulation, increasing myocardial supply and reducing myocardial work.

Although there is progress in drug combination therapy in angina, which most patients begin treatment with nitrates and a beta-adrenoceptor blocking drug or a calcium antagonist, there is a need of drug for chronic stable angina pectoris which is very effective, can be taken for a long period of time and has very low toxicity.

In view of the problems of relating to the above mentioned drugs, notable efforts have been made to apply herbal Medicine as an alternative to the standard treatment of chronic stable angina pectoris. Traditional Chinese Medicine (TCM) has contributed much in this respect.

US Pat. No. 5288485 and 5433957 refer to an extract from *hypericum erectum thunb* for curing or preventing diseases caused by disorder in blood circulation such as angina pectoris.

US Pat. No. 5776463 refers to an oral pharmaceutical composition containing petals of borage or extract of borage petals for the prevention and treatment of stress which is associated with circulatory heart diseases including angina.

In addition to *hypericum erectum thunb* and *borage petals,* many other herbal plants have also been used to treat angina. One such plant is *Valeriana officinalis latifolia.* Yang GY et al (1994) Reported that 82 patients with angina pectoris had been treated with *Valeriana officinalis latifolia,* among whom ST-T ischemic changes appeared on ECG in 50 cases before treatment. Its total effective rate for simple angina (without detectable ischemic findings) was 87.80%; the angina with ischemic findings, 88.00%. In addition, it was discovered that *Valeriana officinalis latifolia* could lower plasma lipids as well. No toxic actions to liver, kidney, and hemopoietic tissue, have been found. (1)

Wu Y (1990) reported that in a setting of 267 patients with angina pectoris, 93.3% of the patients treated with *xintongkang* capsule was effective.

Another herbal preparation called *Shenshao tongguan pian* was used in treating angina pectoris. In 1990, Hu JX et al. Reported that the *Shenshao tongguan pian* is composed chiefly of saponin from the stem and leaves of Ginseng and *Radix Paeoniae Alba,* etc. The total effective rate for treating angina pectoris was 94.71%. And the ECG improvement rate was 63.38%. In addition, laboratory examination also revealed that *Shenshao tongguan pian* could promote the left ventricular output, lower the blood viscosity and inhibit the aggregation of blood platelet. Both acute and chronic toxicity tests showed that *Shenshao tongguan pian* has no toxicity or side effects.

Kuo-guan granule is another herbal preparation for angina pectoris. Li Y et al. (1990) reported that the changes of the plasma zinc, copper and erythrocyte glutathione peroxidase were measured by atomic absorption spectrometry and DTNB color development in 31 patients suffering from coronary heart disease with angina pectoris before and after taking *Kuo-Guan* granule for one month. The results indicated that the plasma zinc and erythrocyte glutathione peroxidase were lower and copper was higher in the patients than the normal control group before treatment (P <0.01), the plasma zinc and erythrocyte glutathione peroxidase increased and copper decreased after treatment (P <0.01). These suggest that therapeutic mechanism of *Kuo-Guan* granule for coronary heart disease with angina pectoris may relate to its regulation on trace elements disturbance in body.

Saponin of *Tribulus terrestris* was another herbal composition for treating angina pectoris.

*Fufang Danshen Pian* is a folk prescription of *Dan Shen* Tablet which indicate for treating chronic stable angina pectoris due to coronary artery ischemia and has been officially listed in the editions of Chinese Pharmacopoeia since 1977 and applied to clinical use for decades.

*Fufang Danshen Pian* contains multiple active extracts of botanical including *Danshen* (*Radix Salviae Miltiorrhizae*), and *Sanchi* (*Radix Notoginseng*). Both of the botanical were first documented in *Shen Nong Ben Cao Jing* (*Shen-nong's* Herbal Pharmacopoeia) completed in 200. *FufangDanshen Pian* also contains synthetic borneol, a version of a natural borneol (Bingpian). Natural borneol was first documented in *TangBen Cao* (Herbal Pharmacopoeia of the Tang Dynasty) compiled around 659.

Li Cheng-zhu et al (1979) reported in (Acta Acad Med Prim Shanghai) on an experimental study of thrombotic inhibition effect of *Radix Silviae Miltiorrhizae.* Effects on *in vitro* thrombosis, functions of platelet and coagulation, fibrinolysis were observed in rabbits. After injection of *Radix Silviae Miltiorrhizae,* 3 links were found to play an important role in inhibition of *in vitro* thrombosis: (1) inhibition of platelet function; (2) inhibition of coagulation function; and (3) promote fibrinolysis. Of which, the former two mechanisms function more intensely. The results conform to those in the treatment of thrombotic diseases, especially arterial thrombotic diseases.

Chiang WT et al (1982) reported in (Acta Acad Med Prim Shanghai) the effects of *"Danshensu"* and other two water-soluble components of *salvia miltiorrhiza* on dog ischemic myocardium and isolated pig coronary artery. The effect of 3 new water-soluble components i.e. *Danshensu* (DS-182, D(+)-3,4-dihydroxyphenyl lactic acid), protocathu-aldehyde (PCAD) and an impure diterpene acid (DS-187) isolated from *Salvia miltiorrhiza Bunge,* were compared with those of dipyridamole. Results revealed (1) in mice, DS-182 gave significant protection against hypoxia, whereas PCAD was ineffective; (2) DS-182 could nullify the pituitrin-induced electrocardiographic ischemic ST-T elevation but had no influence on the reduced heart rate. DS-187, PCAD and dipyridamole only showed incomplete protection; (3) in the acutely infarcted dog model prepared by ligation of the anterior descending branch of the left coronary artery, the benefits achieved by intravenous injection of DS-182 proved superior to DS-187 and PCAD in respect to the left ventricular function, left ventricular peak systolic pressure(LVPSP) and left ventricular end diastolic pressure (LVEDP). PCAD, on the contrary, produced adverse effects on LVPSP and LVEDP. Intravenously administered dipyridamole, though it did not change LVEDP, suppressed LVPSP significantly with marked hypotensive effect. All these components of Salvia and dipyridamole significantly reduced the ultimate myocardial infarct size (N-BT assessment) ; Ds-182 was most effective, dipyridamole and DS-187 the next, while PCAD the least; (4) in the isolated perfused pig coronary artery preparation, DS-182 significantly reduced the resistance of the coronary vessel, whereas either DS-187, PCAD or sodium *Tanshinone* II-A sulfonate (DS-201), another component of *Salvia,* increased it. The constrictory action of morphine and propranolol on the isolated coronary artery preparation was antagonized by the prior administration of DS-182. All of these suggested that *Danshensu* might be the main active principle of *Salvia miltiorrhiza* in treating ischemic heart disease and that its concomitant use with propranolol or morphine would be beneficial.

Li Cheng-zhu et al (1983) reported in (Chin J Integr Med) the anti-coagulation effect of *Radix Silviae Miltiorrhizae. Danshensu* is a water-soluble monomer extracted from *Radix Silviae Miltiorrhizae.* It is also the main ingredient of commercially sold Injection *Radix Silviae Miltiorrhizae.* The present study proved that Danshensu inhibits thrombosis *in vitro,* aggregation of platelet (induced by ADP), and internal and external coagulation systems; diminishes the number of platelets and promotes the degradation of fibrin or fibrinogen. The effects peaked 30 minutes after a single injection of 20 mg/kg in rabbits, lasted for 1 hour, and recovered gradually. 4.5 hours after injection, all recovered to normal but thrombosis test in vitro.

Chen Zhanghua (1987) reported in Acta Acad Med Prim Shanghai on effects of "Danshensu" on experimental microcirculatory disturbances and plasm lactic acid concentrations. Natural *Danshensu* is a water soluble monomer extracted from *Radix Salviae Miltiorrhizae(RSM) .* Microcirculatory disturbances in rabbits were induced by intravenous injection of high molecular weight dextran. Natural *Danshensu* (dosage 4-6 mg/kg) markedly increased the number of capillary vessels in the bulbar conjunctiva, and also decreased the concentration of plasm lactic acid in the rabbits. Mesenteric microcirculatory disorders were produced by local noradrenaline (4 g) drip in mice. Natural *Danshensu* dilated the arteries and accelerated the speed of blood flow, thus eliminating microcirculatory blood stasis. In our experiments, effects of synthetic Danshensu were observed concomitantly. The results showed that there was no significant difference between natural and synthetic *Danshensu* in relieving microcirculatory disturbances.

Sun Xi-ming et al (1991) reported a new pharmacological action of an extract of *Danshen (Salvia miltiorrhiza) .* The paper reports that an extract of *Danshen (Salvia miltiorrhiza)* which contains the sodium salts of D (+) - -(3,4-dihydroxy phenyl) lactic acid was found to possess a new pharmacological action of decreasing the biosynthesis of cholesterol in cells and anti-lipoprotein oxidation, by cell cultural studies. When compared with the control, its electrophoretic migration rate was markedly lowered and MDA content and cytotoxicity decreased obviously. These results indicated that salts of D(+)-(3,4-dihydroxy phenyl) lactic acid may be effective in the prevention and treatment of atherosclerosis.

Zheng Ruo-xuan et al (1992) reported in (Chin J Integr Med) the preservation effect of *Radix Silviae Miltiorrhizae* on myocardial ischemia induced by coronary ligation in mice. Obvious preservation effect on acute myocardial ischemia in mice by coronary ligation could be obtained after i.p. water-extract of *Radix Silviae Miltiorrhizae* (5 g crude drug/kg). S-T segment elevation on ECG due to myocardial ischemia in the treatment group was much lower, ischemic size of the left ventricle was smaller and the survival rate was higher when compared with the control.

Wu Yao-zhong et al (1995) reported in (Acta Nanjing Univ Trad Chin Mater Med) on effects of *Radix Silviae Miltiorrhizae* in promoting blood circulation by removing blood stasis. Pharmacological research of *Radix Silviae Miltiorrhizae* is common. However, rheological studies on *Radix Silviae Miltiorrhizae* by assessing PGI2, ET, and TXA2 produced by platelet are seldom. Influence of *Radix Silviae Miltiorrhizae* on thrombosis, changes of PT, KPTT, FG, ESR and HCT, and aggregation of platelet in rabbits are evaluated in the present study. Conclusions are that *Radix Silviae Miltiorrhizae* reduces the synthesis of TXA2 and decreases the effects of enhancement of platelet aggregation and thrombosis.

Shi Lin et al (1990) reported in (Acta Pharmcol Sin) on the effects of total saponins of *Panax Notoginseng* on increasing PGI₂ in carotid artery and decreasing TXA2 in blood platelet. Total saponins of *Panax notoginseng* (PNS) were given orally 100 mg/(kg·day) to rabbit for 8 wk. Aortic atherosclerotic plaque formation was restrained as compared that of to the control group. Radioimmunoassay was used to investigate the effects of PNS on the contents of prostacyclin in carotid artery and thromboxane A2 in rats' blood platelet. Oral administration of PNS 25, 50, 100 mg/(kg·day) for 10 days caused an increase of prostacyclin in carotid artery and a decrease of thromboxane A2 in blood platelet as compared with the control group. These results showed that the anti-atherosclerotic action of PNS may be a result of the correction of the imbalance between prostacyclin and thromboxane A2.

Li Xing et al (1990) reported in (Acta Pharmacol Sin) the Protective effects of *Panax Notoginseng saponin* on experimental myocardial injury induced by ischemia and reperfusion in rats. Effects of total saponin of *Panax Notoginseng* (PNS) and purified ginsenoids R_{b1} and R_{g1} from PNS on myocardial injury induced by cardiac ischemia and reperfusion were studied using rat hearts in situ and in vitro. In pentobarbital-anesthetized rats, PNS pretreatment (100 and 200 mg/kg) provided significant reduction in myocardial infarcted size after left descending coronary artery ligation (40 min) and reperfusion (120 min) in comparison with the control. PNS 12.5 and 25 mg/L, R_{b1} 10 mg/L and R_{g1} 10 mg/L significantly decreased cardiac CPK release, attenuated myocardial Ca⁺⁺ accumulation, reduced malondialdehyde (MDA) production and prevented reduction of superoxide dismutase (SOD) activity in comparison with the control in perfused isolated rat hearts with global ischemia (40 min) and reperfusion (15 min). The results showed that PNS, R_{b1} and R_{g1} prevented cardiac ischemia and the action was considered to be related to the inhibition of lipid peroxidation.

Huang Cong et al (1991) reported in (Chin Bull Pharmacol)the effects of *Panax Notoginseng Saponin* on myocardial ischemia and reperfusion injury in conscious rabbits. The effects of Panax *Notoginseng saponin* (PNGS) on myocardial ischemia and reperfusion injury in conscious rabbits were studied with observation of changes in electrocardiogram (ECG), the activities of creatine phosphokinase (CPK) and lactate dehydrogenase (LD) and the size of ischemic area. PNGS at the dose of 50 mg/kg and 100 mg/kg significantly reduced the size of myocardial ischemic area. These results suggested that PNGS have the protective effects on myocardial ischemia and reperfusion injury.

Mo Qi-xian et al (1987) reported in (Propriet Trad Chin Med Res) the dynamics of ³H-Borneol. In order to highlight the mechanism of inducing resuscitation of Borneol aromaticity, dynamics of 3H-Borneol were conducted by intraveneous injection and oral administration. The results revealed that the half-life time was 2.8 min after a single intraveneous injection of 3H-Borneol. It suggested that the drug distributed rapidly to the relevent organs and tissues after administration and produced prompt effect. In vivo distribution concentrated on organs and tissues which are abundant in blood flow, such as heart, lung, liver, kidney and brain, etc. This provided clinical application certain theoratical basis. Since the diminishing half-life time was 5.3 hours after oral administration of the drug, this suggested that oral Borneol could not lead to accumulation, but poor bioavailability. Further studies should be taken to discusse the relationship with drug dose and dosage form.

Chen Tie-feng et al (1990) reported in (Acta Pharmacol Sin) the enhancement of absorption of tetramethylpyrazine by synthetic borneol. Sprague-Dawley rats were given ig tetramethylpyrazine phosphate (TMP) 5 mg/kg with or without previous borneol 5 mg/kg. The plasm TMP concentrations were analysed by GC method, and the data were treated by NONLIN program. The Cmax were 931 and 562 ng/ml, respectively, (p<0.01) ; while the AUC were 68849 and 37174, respectively, (P<0.05). It is suggested that the borneol enhances the absorption of the TMP but not in elimination.

Xu Wei et al (1995) reported in (Pharmacol Chin Med Clin) the effect of menthol and borneol on the distribution of sulfadiazine sodium and Evan' s blue in the rat and mouse brain. Menthol (1.5 g/kg, ig) and Borneol (1.5 g/kg) prolonged the sulfadiazine sodium distribution half-life t_{1/2} in rats. The above dosage of menthol and borneol given orally also increased the concentration of sulfadiazine sodium in the rat brain. Menthol (ig 0.5g/kg for 3 days) and borneol (ig 0.5 g/kg for 3 days) promoted the concentration of Evan's blue in the mouse brain, but the value of concentration was significantly lower than that of the mice suffering from the ischemia-reperfusion injury. The results suggested that the menthol and borneol could enhanced the sulfadiazine sodium transfer in brain-blood barrier with no damage to brain-blood barier.

In the United States, coronary atherosclerotic heart disease is the commonest cause of cardiovascular disability and death.

Atherosclerosis is an arterial disorder characterized by yellowish plaques of cholesterol, lipids, and cellular debris in the inner layers of the walls of large and medium-size arteries. The condition begins as a fatty streak and gradually builds to a fibrous plaque or atheromatous lesion. The blood vessel walls become thick, fibrotic, and calcified. The artery lumen narrows.

Many atherosclerotic plaques remain stable or progress gradually. Others may rupture resulting in hemorrhage, platelet activation, and thus intravascular thrombosis. Coronary thrombosis causes partial or complete vessel occlusion, impairs blood flow, thus leads to unstable angina or myocardial infarction. Alternately, the ruptured plaques may become restabilized, often more severe stenosis.

Exercise and mental stresses increase myocardial oxygen demand. Under normal physiological condition, increased myocardial oxygen demand is met by the arterioles dilating thus increasing blood flow. In the presence of atherosclerosis, the arterioles may dilate maximally to meet basic demand. Such dilated arterioles may be unable to meet the increased myocardial oxygen demand. When oxygen demand exceeds oxygen supply, the ischemia of myocardium occurs. Alternately, severe vessel occlusions may limit blood flow thus cause myocardial ischemia. Clinical manifestations of transient myocardial ischemia is angina pectoris which is a paroxysmal thoracic pain, frequently spread to the arms, particularly to the left arm, with or without accompanied by a feeling of suffocating and impending death.

Angina pectoris is subdivided in to two: stable and unstable. Stable angina pectoris is caused by the increased myocardial oxygen demand in most cases. Stable angina thus attacks in the predictable frequency and duration upon provocation which increases myocardial oxygen requirements such as exercise, mental stress, etc. In contrast, unstable angina pectoris attacks without provocation and usually caused by decreased oxygen supply to myocardium. Plaque disruption, platelet plugging, and coronary thrombosis decrease oxygen supply to myocardium.

Angina pectoris is treated with various drugs, surgical procedure, coronary artery bypass graft, balloon-angioplasty, stent placement, etc. Therapy for stable angina pectoris is primarily to minimize myocardial oxygen demand as well as a preventive measure. Therapy for the acute syndrome unstable angina pectoris is primarily to inhibit platelet activation and thrombolysis.

Current therapeutic agents for chronic stable angina pectoris are nitroglycerine, other nitrates, calcium channel blockers, and beta-adrenergic receptor blockers. These drugs, administered alone or in combination with other drugs, alleviate or prevent rather than cure angina.

When angina attacks, nitroglycerine is administered sublingually to alleviate symptoms. Nitroglycerine is also applied to prevent anginal attacks caused by exertion and stress. Nitrates are applied to prevent angina attacks. Nitroglycerine and nitrates mediate their effect primarily by relaxing vascular smooth muscle, reducing myocardial activity, and thus reducing myocardial oxygen demand. The side effects are throbbing headache, dizziness, weakness, orthostatic hypotension, tachycardia, etc.

Beta-adrenergic receptor blockers such as propranolol are applied to prevent angina pectoris by reducing myocardial oxygen requirements during exertion and stress. The major contraindications are bronchospastic disease, bradyarrhythmias, and overt heart failure. In individuals with asthma and other forms of airway obstruction, beta-blockers may worsen their condition.

Calcium antagonists are applied to prevent angina pectoris by reducing the oxygen demand of myocardium. Myocardium is dependent on calcium influx for normal functions. By inhibiting calcium influx, calcium antagonists may relax smooth muscle of the blood vessel, decrease myocardium activity, reduce oxygen demand by myocardium, and thus prevent angina pectoris. Calcium antagonists have adverse side effects. The mild side effects are flushing, edema, dizziness, nausea, etc. Excessive inhibition of calcium influx to myocardium may cause severe side effects such as cardiac arrest, bradycardia, artrioventricular block, congestive heart failure, etc. Combined with beta-adrenergic drugs, the side effects of calcium antagonists are often augmented.

In China, Panax Notoginseng and Radix Salviae Miltorrhizae have been used for treating cardiovascular disease since 200 AD (Shen-nong's Herbal Pharmacopoeia). Panax Notoginseng has been used for treating angina pectoris. Radix Salviae Miltiorrhizae has been used for promoting blood circulation and dispersing blood stasis. Numerous preclinical and clinical studies demonstrate the efficacy and safety of Panax Notoginseng and Radix Salviae Miltiorrhizae.

Traditional Chinese medicine is the mixture of several herbs requiring decoction. A modified form of Chinese medicine for treating coronary heart disease is Dan Shen tablet. Dan Shen tablet is a large unctuous ball, often as large as 1 cM in diameter. Dan Shen tablets are made of the extract of Radix Salviae Miltiorrhizae, powder of Panax Notoginseng and synthetic borneol, have been listed in the Chinese Pharmacopoeia since 1977, and have been used to treat cardiovascular disease for decades.

The disclosed Danshen pill (DSP) or called cardiotonic pill is a new generation Chinese medicine for coronary heart diseases. Chinese medicine consists of various herbs which vary from prescription to prescription in regard to the type of herbs as well as the proportion of herbs. To control the quality, DSP is manufactured with the standarized formula. The therapeutic components of DSP are the water-soluble extracts of Radix Salviae Miltiorrhizae 10-30 % and sometimes, approximately 20 %, the water-soluble extracts of Panax Notoginseng (2-6 %), and borneol (1-3 %). Furthermore, to alleviate angina quickly, DSP been manufactured as a small pill which can be dissolved immediately upon sublingual administration, delivered to myocardium quickly, and thus alleviate angina fast.

DSP has been proven to be nontoxic and effective for the prevention and treatment of cardiovascular disease caused by coronary artery ischemia in preclinical and clinical studies. Furthermore, the superior efficacy of DSP to Dan Shen tablets for treating coronary arterial disease has been demonstrated in preclinical as well as in clinical studies.

DSP has been listed in the Supplement Edition of Chinese Pharmacopoeia since 1998, approved by the Chinese Ministry of Health, marketed as a drug in China since 1993, and used by more than five million people.

### SUMMARY OF THE INVENTION

The disclosed Dan Shen Pill (DSP) is a new generation of Chinese medicine for treating coronary heart disease manufactured with the standarized formula. DSP comprises the standarized amount of the extracts of Panax Notoginseng, the extracts of Radix Salviae Miltorrhizae, and Borneol.

Panax Notoginseng is included to alleviate and to prevent angina. Radix Salviae Miltorrhizae is included to inhibit platelet activation, to prevent coronary thrombosis, and thus to promote blood circulation. Borneol is included for the effective delivery of therapeutic components to myocardium.

DSP is manufactured as a small pill, approximately 25 mg, which can be dissolved immediately upon sublingual administration and thus to mediate its therapeutic effects quickly. The efficacy of DSP alleviating and preventing angina has been proven in preclinical and clinical studies.

The present invention discloses a process for preparing a Dan Shen pill (DSP) for use in treating chronic stable angina pectoris and a DS pill obtainable by this process as set forth in the appended claims

### DETAILED DESCRIPTION OF THE FIGURES

**Figure 1****.** Batch No.: 19990806
**Figure 2****.** Batch No.: 19990815
**Figure 3****.** Batch No.: 19990823

The position of fingerprint peaks: Group I consisting of peak 1 and 2 (retention time ranging from 7 to 15min) ; Group II consisting of peak 3 and 4 (retention time ranging from 15 to 20min) ; Group III consisting of peak 5, 6, 7 and 8 (retention time ranging from 20 to 40min). From whole sight, the abundance of peaks in the group I is largest, and that the height of peak 1 is close to the peak 2. The peaks in the group II stand side by side, posses same heights almost and the abundance is very small. The group III is composed of 4 peaks, and their height increase step by step. These three groups compose the representative fingerprint of Compound Danshen Dripping pills.

### DETAILED DESCRIPTION OF THE INVENTION

This invention refers to a composition of the medicament Dan Shen Pill (DSP) comprising:
(a) [water-soluble] extracts of Radix Salviae Miltorrhizae, [water-soluble] extracts of Panax Notoginseng, and [synthetic] Borneol;
(b) Radix Salviae Miltorrhizae, Notoginseng, borneol, and carriers; and
(c) Radix Salviae Miltorrhizae, Panax Notoginseng, borneol, and pharmaceutical carriers.

In an embodiment of the above medicament, Radix Salviae Miltorrhizae and Panax Notoginseng are employed as they are used in China for treating coronary heart disease since 200 AD. Panax Notoginseng has been used to treat angina and Radix Salviae Miltorrhizae has been used to promote blood circulation. Borneol is employed to facilitate the fast delivery of therapeutic components to target organs. Natural Borneol has been used in China since 600 AD. As Borneol is almost extinct, DSP comprises synthetic borneol.

This invention provides the use of DSP for:
(a) treating coronary heart disease;
(b) treating coronary heart disease in conjunction with other drugs;
(c) primary prevention of coronary heart disease;
(d) primary prevention of coronary heart disease in conjunction with
   other drugs;
(e) secondary prevention of coronary heart disease;
(f) secondary prevention of coronary heart disease in conjunction with other drugs; and
(g) reducing nitrates intake by angina patients; and
(h) reducing serum cholesterol level.

In an embodiment of DSP, the indication is for coronary artherosclerotic disease such as, but not limited, to alleviate angina pectoris, to prevent angina pectoris caused by exertion and stress, and to promote blood circulation by inhibiting platelet aggregation thus to preventing coronary thrombus formation. DSP can be applied to reduce nitroglycerine intake which is frequently used to alleviate and to prevent angina. DSP can be also applied to reduce plasma cholesterol level thus to prevent the formation of new atherosclerosis lesions. Atherosclerosis is often initiated by cholesterol streak deposited on vessel walls.

In another embodiment, DSP comprises 5-40% water-soluble phenolic acid of Radix Salviae Miltorrhizae, 1-10 % water soluble saponin of Panax Notoginseng, and 1-5% borneol.

In another embodiment, DSP comprises 10-30 % water-soluble phenolic acid of Radix Salviae Miltorrhizae, 2-6 % water soluble saponin of Panax Notoginseng, and 1-3 % borneol.

In another embodiment, the size of small pills are:
(a) 0.33-0.34 cm in diameter;
(b) 21.25-28.75 mg in weight; and
(c) 1.13-1.40 mg/mm³ in density.

### EXPERIMENTAL DETAILS

### Manufacturing of Dan Shen Pill (DSP)

DSP is a small pill, approximately 25 mg, of which therapeutic components comprise water-soluble extracts of Notoginseng, water soluble extracts of Salviae, and synthetic borneol.

For manufacturing DSP, Notoginseng and Salviae are extracted separately with hot water in circulating systems and filtered. The filtrates are condensed under decompressed conditions, filtered and precipitated. The concentrates are refined using resin columns and concentrated under decompressed conditions. The refined water-soluble extracts thus obtained were mixed with synthetic Borneol and pharmaceutical carriers. The mixture is made to a small pill using a special dropping machine. The quality of DSP is controlled by standarizing the quantity as well as the proportion of its major therapeutic components Saponon, phenolic acid such as Danshensu and Borneol. Thin-layer chromatography, high performance liquid chromatography, fingerprinting and other analytical techniques are used to identify and quantitate therapeutic components in DSP.

### Manufacturing of DSP

1. Extraction of water-soluble components of Panax Notoginseng
   (a) Dilution of herbs with 5-7 times of water.
   (b) Extraction of water-soluble components of Panax Notoginseng by boiling in a tank with the air pressure between 0.04-0.06 mPa for 2 hours.
   (c) Repeat extraction under the same condition for 1.5 hours.
   (d) Filtration of the extraction with 100-mesh net.
   (e) Refine the filtrate using macroporous adsorption resin eluting with ethanol.
   (g) Concentration of the eluted extracts under decompressed condition with the air input to 0.04-0.06 mPa and the vacuum to -0.076~-0.088 mPa until the density is 1.33-1.35.
2. Extraction of water-soluble components of Radix Salviae Miltorrhizae
   (a) Dilution of herbs with 5-7 times of water.
   (b) Extraction of water-soluble components of Radix Salviae Miltorrhizae by boiling in a tank with the air pressure between 0.04-0.06 mPa for 2 hours.
   (c) Repeat extraction under the same condition for 1.5 hours.
   (d) Filtration of the extraction with 100-mesh net.
   (e) Concentration of the filtrates under decompressed conditions with the vacuum pressure is-0.076--0.088 mPa until one Kg initial herb becomes one liter.]
   (f) Precipitation of the concentrates with ethanol.
   (g) Filtration of the ethanol precipitates solution through 100-mesh net.
   (h) Concentration of the filtrates under decompressed conditions with input air pressure is 0.04-0.06 mPa and the vacuum pressure is 0.076~-0.088 mPa.
   (i) Refine the concentrates by polyamide chromatography eluting with ethanol.
   (j) Concentrate the refined extracts to the density of 1.33-1.35.
3. DSP production
   (a) Mix the extracts of Panax Notoginseng, the extracts of Radix Salviae Miltorrhizae, synthetic boneol and polyethylene glycol 6000 at the ratio of 4.0:20.6:1.9:79.5.
   (b) Melting the mixture.
   (c) Manufacturing the melted mixture to DSP using the dropping machine with the following characteristics: the temperature of dropping pot is constantly 89-93°C, the cooling solution is liquid paraffin of which the temperature is lower than 8°C, the inner diameter of the dropping head is 1.8mm, the outer diameter of the dropping head is 2.4mm, the distance between the dropping head and the surface of cooling solution is 15 cm.
   (d) Centrifugation of the pills at 800-1100 rpm for 15 minutes to remove oils.

### Quality control of DSP

DSP contains the identified therapeutic components protocatechuic aldehyde and saponin as well as various other components. The contents of these compounds in herbs vary from lot to lot of herbs. To standarize the contents of therapeutic components in DSP and thus to control the quality of DSP, a method to identify and to quantitate therapeutic agents in medicaments has been developed. An example of the procedures comprises:
(a) Dissolve 30 DSPs in 3 ml methanol and ultrasonicate for 10 minutes.
(b) Centrifugation for 5 minutes.
(c) Fractionate the supernatant using standard analytical techniques using thin layer chromatography, high performance liquid chromatography, etc.
(d) Identification of the therapeutic components of DSP such as Sodium Danshensu, protocatechuic aldehyde, saponin, etc. by comparing DSP fractions with the relevant purified standards in regard to the position, size and color.
(e) Identifying and quantitating therapeutic components in DSP by comparing the position, size, and color of DSP fractions with the position, size and color of the relevant purified standards.
(f) Identifying therapeutic components in DSP by comparing the relative retention time and relative area of peaks in fingerprints with the relative retention time and relative area of peaks in standard fingerprint.

### TLC identification of DSP

A method for identifying Sodium *Danshensu* and protocatechuic aldehyde of a herbal composition capable of treating chronic stable angina pectoris by thin layer chromatography comprising the steps of:
a) preparing the assay comprising the steps of:
   i. putting 30 pellets of the said composition in 3 ml methanol and dissolve by ultrasonation for 10 minutes to form a solution;
   ii. centrifuging the solution for 5 minutes and collect the supernatant;
   iii. contacting 10 ul the solution onto a silicon G gel plate containing 0.5% CMC-Na;
   iv. developing the plate with a developing solution comsisting of Chloroform, acetone and methane acid in the ratio of 10:4:1.6;
   v. drying and fumigating the plate with ammonia and laying the plate up for 15 minutes;
   vi. checking the plate under ultraviolet light, the spot representing the said composition should be at the corresponding position of the standards and show the same color.
b) using Sodium *Danshensu* and protocatechuic aldehyde as the standards.

A method for identifying gypenoside of a herbal composition capable of treating chronic stable angina pectoris by thin layer chromatography comprising the steps of:
a) preparing the assay comprising the steps of:
   i. Put 30 pellets of the said composition in 5 ml ammonia solvent and dissolve by ultrasonation to form a solution;
   ii Put the said solution into the macroporous adsorption resin column; the speed is 0.5/minute;
   iii. After washing the macroporous adsorption resin column with 20 ml distilled water, the macroporous adsorption resin column is eluted with 2 ml methanol solution;
   iv. collecting the eluant;
   v. contacting 10 ul said eluant onto a silicon G gel plate containing 0.5% CMC-Na;
   vi. developing the plate with 10 ml developing solution which is a lower layer clarificant of the solution of Chloroform, acetone and water in the ratio of 6:3:1 after 2 hours at 10□C;
   vii. After being dried and sprayed with 10% ethanol sulfate, the plate is baked at 105□C for several minutes;
   viii. Check the plate under normal light, the spot representing the said composition should be at the corresponding position of the standards and show the same color.
b) using total gypenoside, Saponin R1 and ginsenoside Rg1 as the standards.

### Fingerprints of Cardiotonic Pill

### 1. Preparation of fingerprints

### (1) Chromatographic system and system suitability

Alkyl silan-linking silico-18 was used as the stationary phase, and the mixture of A and B as mobile phase. A was methanol and B was the mixture of water- "N,N-dimethyl-formamide" -glacial acetic acid (100: 45: 4). The concentration of A changes from 5% to 30% when time of gradient elution elapses from 0 to 25 minutes. The detective wavelength was set at 281nm. The number of theoretical plates of the column was not less than 2000 when calculated with the peak of Danshensu.

### (2) Apparatus and reagents

Chromatograph: HP 1100 Liquid Chromatograph
Detector: HP VWD-stile ultraviolet detector
Column: Alltech Company 5u, 250×4.6mm, ODS column
Pre-column: Alltech Company, Alltima C₁₈ 5u pre-column
Temperature of the column: 30°C

### (3) Preparation of the control sample

Salvianic acid B, Danshensu and protocatechuic aldehyde were dissolved respectively in methanol to produce three control solutions each ml containing 50 µ g, 40 µ g and 10 µ g correspondingly.

### (4) Preparation of the test sample

Put 10 pills of Cardiotonic Pill into a 25 ml measuring flask, add 20ml of methanol, ultrasonicate for 20 minutes, allow to cool, dilute the solution to the scale-line with methanol, centrifugate and take the supernate as the test solution.

### (5) Procedure

Accurately inject 10 µl each of the control solutions and the test solution, respectively, into the column, record the chromatograph chart and calculate the content.

### 2. Fingerprints of different batches of Cardiotonic Pill

Twenty batches of Cardiotonic Pill samples were tested with the above-described method, and statistical data is shown as **Table 1, Table 2** and **Table 3.** These data revealed that Cardiotonic Pill had specific fingerprint of its own and the fingerprints contained eight common peaks, that is, these peaks should exist simultaneously in each batch of DSP. Taking protocatechuic aldehyde peak as reference peak, whose relative retention time was 1, the average value of relative retention time of the eight peaks was 0.672, 1.000, 1.417, 1.512, 2.016, 2.235, 2.407, 2.757. The values of relative retention time and relative area of the eight peaks were very stable, and among the eight common peaks, peak No.1 was Danshensu, peak No.2 protocatechuic aldehyde and peak No.7 Salvianic acid B, and the ratio of relative area value was 0.476-0.668: 1: 0.391-0.641, respectively.

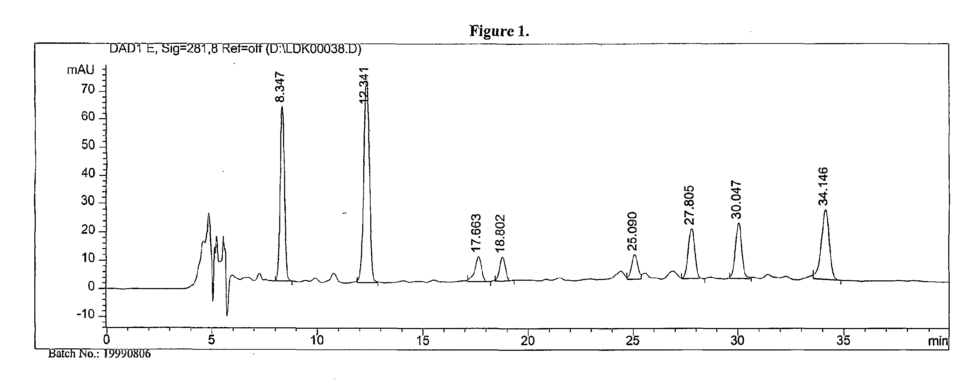

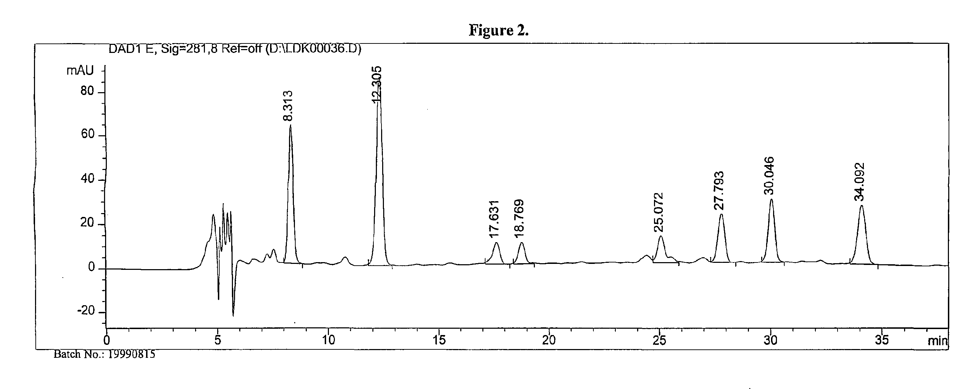

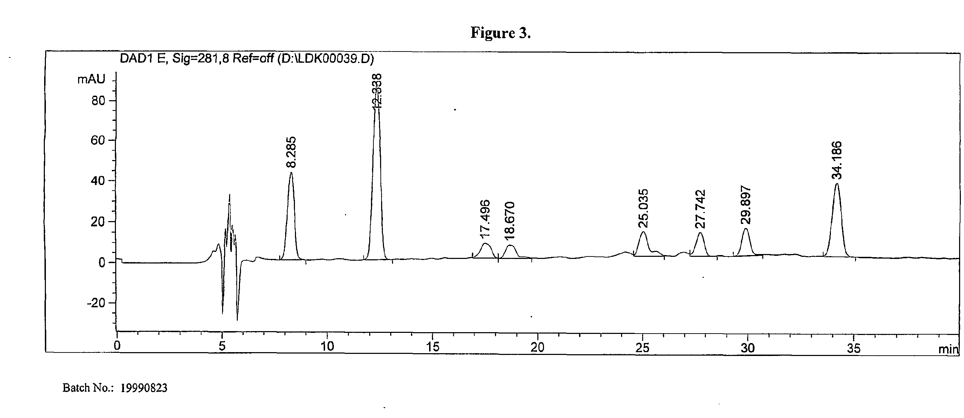

Fingerprints of three batches of Cardiotonic Pill are also provided as **Figures 1** through **3**.

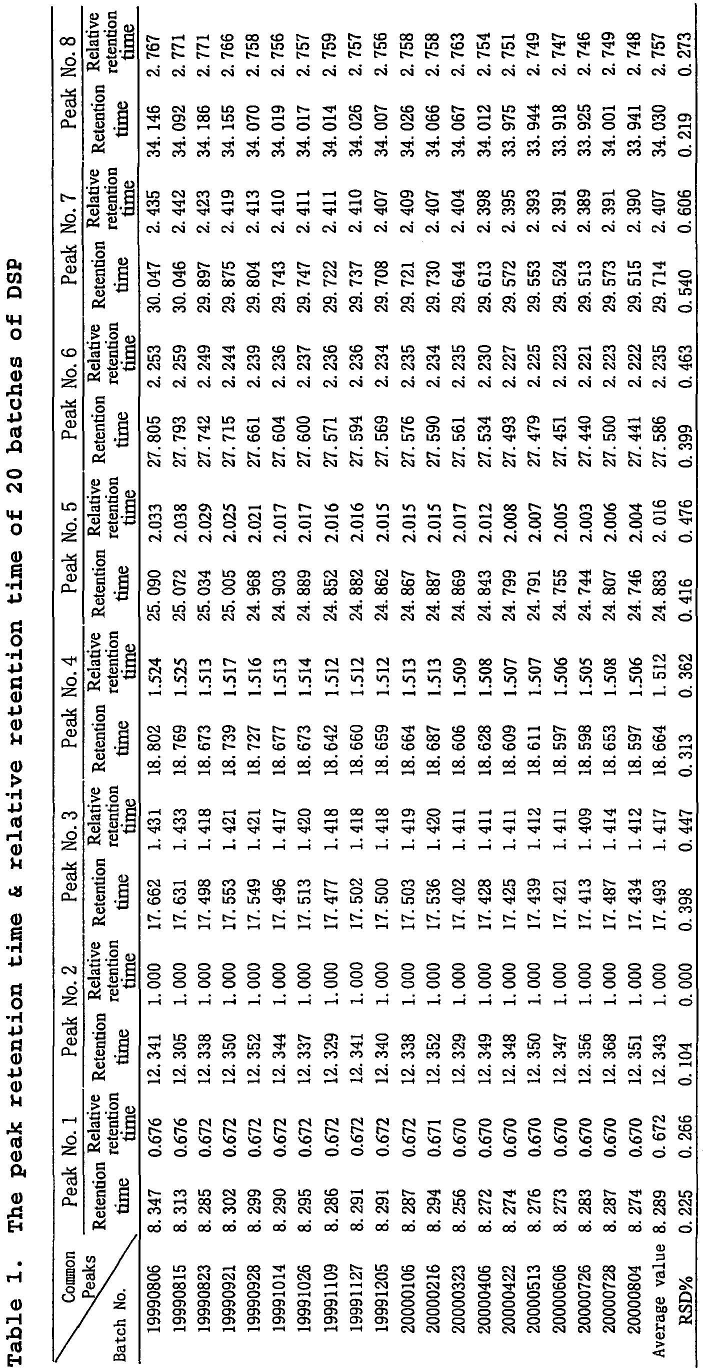

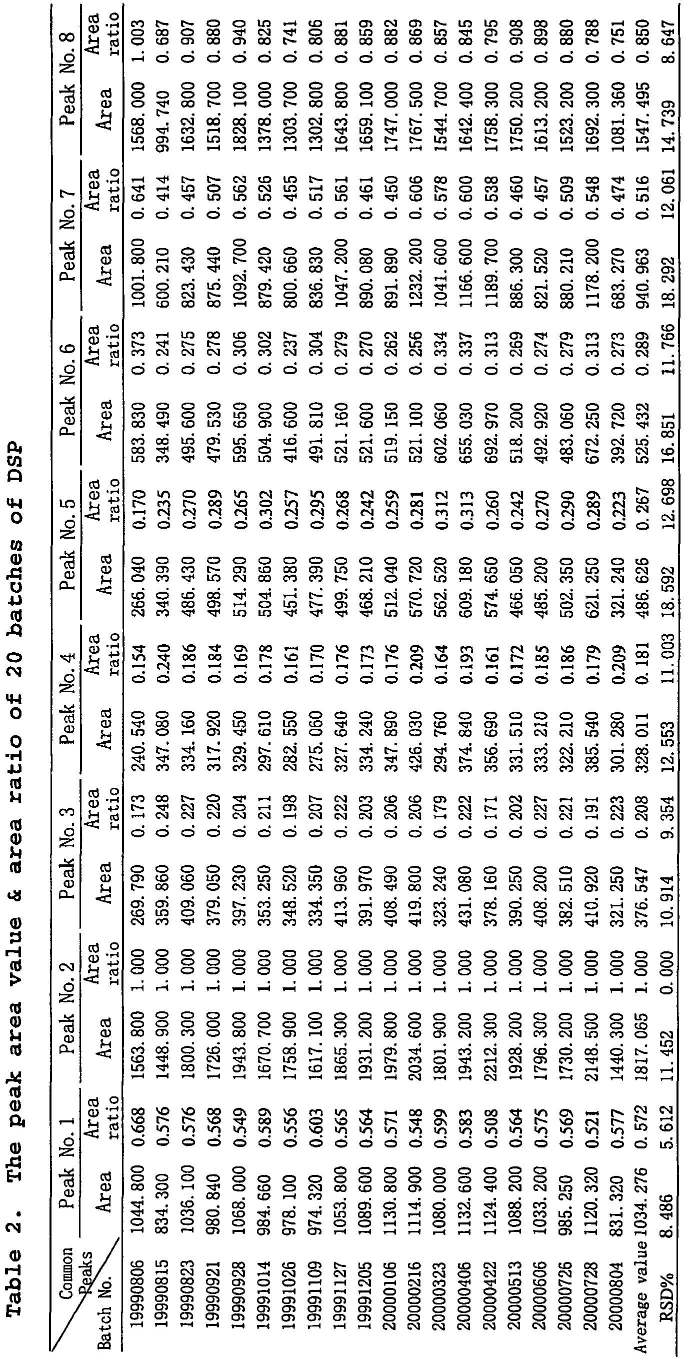

**Table 3. The statistical data of eight peaks derived from fingerprints of 20 batches of DSP**

| Peak No. | Retention Time | Relative Retention Time | Appearance Probability | Area | Area Ratio | Area Ratio Range |
|---|---|---|---|---|---|---|
| 1 | 8.289 | 0.672 | 100% | 1034.276 | 0.572 | 0.572±0.096 |
| 2 | 12.343 | 1.000 | 100% | 1817.065 | 1.000 | 1. 000±0.000 |
| 3 | 17.493 | 1.417 | 100% | 376.547 | 0.208 | 0.208±0.040 |
| 4 | 18.664 | 1.512 | 100% | 328.011 | 0.181 | 0.181±0.059 |
| 5 | 24.883 | 2.016 | 100% | 486.626 | 0.267 | 0.267±0.097 |
| 6 | 27.586 | 2.235 | 100% | 525.432 | 0.289 | 0.289±0.052 |
| 7 | 29.714 | 2.407 | 100% | 940.963 | 0.516 | 0.516±0.125 |
| 8 | 34.030 | 2.757 | 100% | 1547.495 | 0.850 | 0.850±0.163 |

### Quantitative analysis of Danshensu in DSP

Chromatography and systemic adaptive conditions, apparatus and reagents:

### 1. Preparation of fingerprints

### (1) The parameters of Chromatogram & system adjustment.

Alkyl silan-linking silico-18 was used as filling material, and water-acetonitrile-glacial acetic acid (87: 12: 1) as mobile phase. Detective wave length was set at 281 nm. The number of theoretical plate should not be less than 2500 when calculated with the peak of Danshensu, and the degree of separation should meet the requirements.

### (2) Apparatus & reagents

Chromatograph: HP 1100 Liquid Chromatograph
Detector: HP VWD-stile ultraviolet detector
Column: Alltech Company 5u, 250 x 4.6 mm, ODS column
Pre-column: Alltech Company, Alltima C18 5u pre-column
Temperature of the column: 30°C
Acetonitrile: chromatographically pure, Tianjin Siyou Biomedical & Technical Co. Ltd.
Glacial acetic acid: analytically pure, Tianjin Tianhe Reagent Company.

### (3) Preparation of the control sample.

Use 25.0 mg of sodium salvianic acid and 5.0mg of protocatechuic aldehyde as the control samples: Weigh both of the samples accurately and put them into the 50 ml measuring flasks. Add mobile phase to dissolve them and dilute the solutions up to the scale-line of the flasks, shake them thoroughly and save them as the stock solutions. Weigh a little amount of paraaminobenzoic acid accurately, dissolve it as a solution of 0.2 mg/ml with the mobile phase and take it as the internal standard stock solution. Pipit proper amounts of sodium salvianic acid A, protocatechuic aldehyde and internal standard solutions whit their volumes accurately read, dilute them with the mobile phase to prepare a solution that contained 50 ug of sodium salvianic acid A, 10 ug of protocatechuic aldehyde and 80 ug of paraaminobenzoic acid. The prepared solution was taken as the control solution.

### (4) Preparation of the test sample.

Take 10 pills of Cardiotonic Pill and 1 ml of internal standard stock solution, put them into a 25 ml measuring flask, dissolve them with mobile phase, and dilute the solution to the scale-line.

Take 10 ml of the control and the test sample solutions, respectively, make the injection and record the chromatograph chart.

Preparation of the control solution: Take and weigh accurately 25.0mg of sodium tanshinol, and put it into a measuring flask. Add the mobile phase, and dissolve and dilute it to the scale. Shake the solution up, and keep it as the control stock solution. Weigh para-aminobenzoic acid accurately and dilute it into a 0.2mg/ml solution with the mobile phase. Keep the solution as the internal standard stock solution. Take appropriate doses of the control stock solution and the internal standard stock solution, and make them into the control solution comprising 50 µg of sodium tanshinol and 80µg of para-aminobenzoic acid per milliliter.

Preparation of the test solution: Take 10 pills of this article and 1ml of the internal stock solution. Put them in a 25ml measuring flask, dissolve them to the scale and make them into the test solution.

Take 10l of the control solution and 101 of the test solution respectively, take down the fingerprints and calculate the results.

The herbal composition comprising DSP should contains 0.14-0.18mg Danshensu per pill.

### Quantitative analysis of Ginsenoside Rg1 and Sanchinoside R1 in DSP

### (1) Chromatographic system and system suitability

Alkyl silan-linking silico-18 was used as the stationary phase, and the mixture of water and acetonitrile as mobile phase. The concentration of acetonitrile was 25% from 0 to 15 minutes, and 35% after 15th minute. Nebulizer gas flowrate was 2.5 liter per minute and drift tube temperature was set at 93.8°C. The number of theoretical plates of the column was not less than 5000 when calculated with the peak of Ginsenoside Rg1.

### (2) Apparatus and reagents

Chromatograph: Agilent 1100 Liquid Chromatograph
Detector: Alltech ELSD 2000 detector (evaporative light scattering detector)
Column: Alltech Company 5u, 250×4.6mm, ODS-C₁₈ column
Pre-column: Alltech Company, Alltima C₁₈ 5u pre-column
Temperature of the column: 30°C

### (3) Preparation of the control sample

Ginsenoside Rg1 and Sanchinoside R1 were dissolved respectively in methanol to produce two control solutions each ml containing 0.98mg and 0.25mg correspondingly.

### (4) Preparation of the test sample

Put 50 pills of Cardiotonic Pill into a 5 ml measuring flask, add 4% ammonia to the scale-line, ultrasonicate for 20 minutes, and apply the solution to a previously prepared small C₁₈ column (STRATA C18-E column of Phenomenex Company, 500mg and 3cc tube), elute 10ml of water, discard the eluate, then elute 2ml of methanol, collect the eluate in a measuring flask and dilute it to the scale-line with methanol, take the solution as the test solution.

### (5) Procedure

Accurately inject 10µl each of the control solutions and the test solution, respectively, into the column, record the chromatograph chart and calculate the content.

### (6) Result

Twenty batches of Cardiotonic Pill samples were tested with the above-described method, and statistical data is shown as **Table 4**. Drawn on above table, the herbal composition comprising DSP contains 0.401%-0.712%, average 0.550% Sanchinoside R1 and 2.069%-4.044%, average 2.847% Ginsenoside Rg1.

**Table 4. Quantities of Ginsenoside Rg1 and Sanchinoside R1 in DSP**

| Batch No. | Sanchinoside R1 (µ g/pill) | Ginsenoside Rg1 (µ g/pill) |
|---|---|---|
| 20000106 | 17.22 | 80.93 |
| 20000216 | 16.92 | 80.78 |
| 20000323 | 15.16 | 70.76 |
| 20000406 | 13.65 | 62.51 |
| 20000422 | 14.24 | 68.72 |
| 20000513 | 15.27 | 71.16 |
| 20000606 | 14.86 | 68.21 |
| 20000726 | 14.59 | 72.35 |
| 20000728 | 14.25 | 57.37 |
| 20000804 | 15.30 | 70.55 |
| Average value | 15.15 | 70.33 |
| RSD% | 7.53 | 9.67 |
| not less than | 12.12 | 56.26 |

The characteristics of DSP thus manufactured are as follows:
(a) DSP contain b-3,4-dihydroxyphenyl lactic acid, sodium danshensu, saponin, and borneol,
(b) negative for bacteria: contains less than 1,000 bacteria,
(c) negative for fungi: contains less than 100 fungi,
(d) negative for heavy metal: contains less than the safety amount defined by the Chinese government.
(e) The shelf-life of DSP is four years at room temperature.

### CLINICAL STUDIES OF DSP

Angina pectoris is evaluated by the history of angina, serum lipid level, electrocardiography (ECG), exercise ECG, scintiographic assessment of ischemia, coronary angiography, etc. Assessing therapeutic efficacy using these end points, DSP has been shown to be effective for treating angina pectoris.

### DSP is effective for angina pectoris.

157 patients with coronary heart disease were treated with 10 DSPs per t.i.d., oral administration for 4 weeks. Assessing the frequency, intensity and duration of angina, oppressed feeling in chest and palpitation, the symptoms were disappeared or remitted in 95.3% patients.

### DSP is more efficient than Dan Shen tablets in alleviating angina.

Dan Shen tablet is another Chinese medicament for treating angina pectoris currently used in China. The efficacy of DSP and Dan Shen tablet was compared. Coronary heart disease patients were randomly divided into two groups. 107 patients were treated with DSP and 50 patients were treated with Dan Shen Tablet. Comparing the frequency of angina attacks and the consumption of nitrates, DSP was more effective than Dan Shen Tablets for treating angina pectoris. See **Table 5** below.

**Table 5. Comparison of DSP and Dan Shen tablet**

| Number of patients | | |
|---|---|---|
| Total | | Responsive |
| DSP | 107 | 102 (95.3%) |
| Dan Shen tablet | 50 | 38 (76%) |

Having proven that DSP is more effective than Dan Shen tablets, the efficacy of DSP was compared with various drugs which are currently used for treating chronic stable angina pectoris in the US.

### Comparison of DSP and nitroglycerine

Nitroglycerine is the frequently used to relieve angina. The efficacy of DSP and nitroglycerine relieving angina was compared. At the onset of angina, patients were treated with either DSP or nitroglycerine, and the time required to alleviate angina was compared. Both DSP and nitroglycerine alleviate angina in all patients within 15 minutes. The efficacy of DSP was slightly lower than nitroglycerine. See **Table 6** below.

**Table 6. Comparison of DSP and nitroglycerine**

| # patients responded | | | |
|---|---|---|---|
| within 1-5 minutes 6-10 minutes 11-15 minutes | | | |
| DSP | 11 | 14 | 5 |
| Nitroglycerine | 17 | 12 | 1 |

| | | | |
|---|---|---|---|
| Total: 30 patients per group | | | |

### DSP does not change heart rate

The data demonstrate that DSP effectively alleviate angina. It was examined whether DSP relieves anginal by increasing heart rate. The heart rate after DSP treatment was equivalent to the pretreatment rate, which indicates that DSP relieves angina without affecting heart rate (**Table 7**).

**Table 7. DSP does not affect heart rate**

| | Heart rate | |
|---|---|---|
| | Pretreatment | Post-treatment |
| DSP | 84.3 ± 23.1 | 82.8 ± 22.8 |

### Comparison of the efficacy of DSP with the nitrate isosorbide dinitrate

Having proven that DSP alleviate angina as efficiently as nitroglycerin, it was examined whether DSP can prevent angina. The efficacy of DSP and the nitrate isosorbide dinitrate was compared. Isosorbide dinitrate is a long-acting nitrate frequently used for preventing chronic stable angina pectoris in the US. Patients were treated with either DSP three times per day orally 10 pills per treatment or Isosorbide dinitrate three times per day orally 10 mg per treatment. Cardiac function and Electrocardiogram was examined.

### Cardiac function.

The efficacy of DSP and nitrates on cardiac function was evaluated by measuring cardiac output per stroke (CO), stroke volume per minute (SV), eject blood fraction (EF), fraction of shortened rate of left ventricular short axis (FS). DSP improves cardiac function more efficiently than nitrates. See **Table 8** below.

**Table 8. The effect of DSP and nitrates on cardiac function**

| | DSP | | Isosorbide initrate | |
|---|---|---|---|---|
| | Pre- | Post- | Pre- | Post |
| SV | 75.38 ± 8.32 | 83.45 ± 9.11 | 74.96 ± 8.44 | 79.47 ± 8.72 |
| CO | 5.61 ± 1.34 | 6.94 ± 1.36 | 6.54 ± 1.36 | 6.12 ± 1.41 |
| EF | 0.57 ± 0.02 | 0.79 ± 0.02 | 0.59 ± 0.03 | 0.70 ± 0.03 |
| FS | 17.14 ± 3.4 | 16.69 ± 3.6 | 17.32 ± 3.1 | 18.46 ± 4.2 |

| | | | | |
|---|---|---|---|---|
| Pre- : preteatment Post-: post-treatment | | | | |

### DSP improves ECG

The ST-T effective rate was evaluated by recording the frequency of change in the ST-T segment. Both DSP and isosorbide dinitrates decreased the frequency of change in ST-T significantly. DSP, however, was more efficient. See **Table 9** below.

**Table 9. Comparison of the effect of DSP and nitrate on ECG**

| The frequency of ST-T change | | |
|---|---|---|
| | Pretreatment | Post-treatment |
| DSP | 131 | 35 |
| isosorbide dinitrate | 129 | 42 |

### Comparison of DSP and aspirin in reducing blood stasis

Hemorrhage at the atherosclerotic lesions induces platelet activation, coronary thrombosis and blood stasis resulting in impaired blood flow. Thus to improve blood flow, chronic administration of the platelet-activation inhibitor aspirin is recommended for patients with angina. The efficacy of DSP and aspirin improving blood flow was compared by evaluating [Hb, Lb, P and air flow] . DSP improves blood flow as efficiently as aspirin. DSP improves blood flow as efficiently as aspirin. See Table 10 below.

**Table 10. DSP and aspirin improve blood flow**

| | DSP | | aspirin | |
|---|---|---|---|---|
| | Pre- | Post- | Pre- | Post- |
| Hb | 6.23 ± 1.67 | 4.35 ± 1.02 | 6.12 ± 1.56 | 4.28 ± 1.07 |
| LB | 10.92 ± 2.21 | 8.30 ± 1.14 | 10.38 ± 1.96 | 8.21 ± 0.3 |
| P | 1.95 ± 0.08 | 1.77 ± 0.08 | 1.89 ± 0.12 | 1.67 ± 0.7 |
| Air flow | 1.79 ± 0.13 | 1.39 ± 0.11 | 1.82 ± 0.17 | 1.40 ± 0.10 |

| | | | | |
|---|---|---|---|---|
| Total number of patients: 25 for DSP and 28 for aspirin | | | | |

### DSP reduces blood stasis by inhibiting platelet activation

Thromboxane B2 activates platelet. Activated platelets release various substances including β platelet microglobulin, which causes blood stasis, thus impairing blood flow. The efficacy of DSP inhibiting platelet activation was examined. DSP lowers thromboxane B2 concentration and inhibits platelet aggregation efficiently. Isosorbide dinitrate, which is known to be unable to inhibit platelet activation thus used as control, did not reduce thromboxane B2 or inhibit platelet activation. See **Table 11** below.

**Table 11. DSP inhibits platelet activation**

| | | Pretreatment | Post-treatment |
|---|---|---|---|
| β PM | DSP | 62.44 ± 14.37 | 45.65 ± 12.25 |
| | nitrates | 59.89 ± 15.42 | 54.36 ± 13.18 |
| Txβ2 | DSP | 1312 ± 535 | 738 ± 384 |
| | nitrates | 1315 ± 507 | 1218 ± 445 |

### DSP lowers plasma cholesterol level

Increased plasma cholesterol has been implicated in the initiation of atherosclerosis. To prevent the formation of new atherosclerotic lesions, the decrease of plasma cholesterol either by modifying diets or drugs was recommended. It was examined whether DSP decreases plasma cholesterol. DSP lowered the cholesterol level by 0.3 mmol/L, which is statistically significant at the P value 0.05. See **Table 12** below.

**Table 12. DSP decreases plasma cholesterol**

| | Plasma cholesterol (mmol/L) | |
|---|---|---|
| | Pretreatment | Posttreatment |
| DSP | 5.15 ± 0.16 | 4.84 ± 0.2 |

### Number of patients: 80

### Cardiotonic Pill's Effect on LPO and SOD in the Blood Serum of Patients Suffering from Coronary Heart Disease

The method: In the treatment group, 24 patients, in accordance with China Reference Diagnosis Standards for Coronary Heart Disease amended in 1979, are administered with Cardiotonic Pill, 10 pills/time, and 3 times/day. In the normal group, 20 healthy people do not receive any medical treatment.

The results: The level of LPO of the patients suffering from coronary heart disease is clearly higher than that of the healthy people, while the content of SOD is clearly lower (p<0.01). After the patients are treated with Cardiotonic Pill, their LPO evidently decreases (p<0.01), and their SOD evidently increases (p<0.01). See **Table 13** below.

**Table 13. A Comparison of the Contents of SOD and LPO in the Normal and the Treatment Groups (x±s)**

| Groups | Patients | SOD (ng/ml) | | LPO (nmol/ml) |
|---|---|---|---|---|
| Normal group | 20 | 348±106 | | 4.64±1.52 |
| Treatment group | 24 | Before treatment | 267±76* | 7.16±1.48* |
| | | After treatment | 309±87#. | 4.68±1.72## |

| | | | | |
|---|---|---|---|---|
| Note: In comparison with the normal group, *p<0.05. In comparing with those of pre-treatment, #p<0.05,##p<0.01. | | | | |

The conclusion: Chronic ischemia of cardiac muscles of patients suffering from coronary heart disease and tissue anoxia lead to the reduction of the activity of SOD, especially that of extra cellular SOD, and the increase of oxygen free radicals, which causes the elevation of LPO and consumption of SOD further. After the patients are treated with Cardiotonic Pill, the level of LPO decreases evidently, while the content of the SOD increases obviously. This proves that Cardiotonic Pill has a strong action of clearance on oxygen free radicals, which is also one of the mechanisms to treat coronary heart disease.

### The Effect of Cardiotonic Pill on the Activities of LPO and Antioxidases in Treatment of Pulmonary Heart Disease

The method: The subjects: 48 patients suffering from pulmonary heart disease are randomly divided into 3 groups. In the normal group, 16 patients are treated with a complex of therapies, such as anti-inflamatory therapy, antiasthma, oxygen inhalation, and so on. In the Cardiotonic Pill group, 14 patients are treated with Cardiotonic Pill, 10 pills/time, and 3 times/day. In the Gantangzhi group , 18 patients are treated with the intravenous drip----200mg of Gantangzhi dissolved in 250ml of 5% glucose injection, 1 time/day, and 10 days/period.

The results: After treatment with Cardiotonic Pill, the value of GSH-Px goes up, the value of LPO goes down, and, therefore, GSH-Px/LPO goes up. In comparison with the normal treatment group, there is a significant difference. See **Table 14** below.

**Table 14. Indices before and after Treatments**

| | | GSH-Pₓ (U/mgHb) | CAT (U/gHb) | SOD (U/gHb) | LPO (nmol/ml) | GSH-Pₓ/LPO |
|---|---|---|---|---|---|---|
| Healthy people group | | 140.6±35.2 | 312.7± 58.1 | 5799.8±948 | 4.2±1.2 | 34.2±8.7 |
| Normal group | Before treatment | 101.3±23.6 | 300.4± 107.7 | 5740.5 ± 939.0 | 5.6±1.9 | 20.4±8.8 |
| | After treatment | 120.6± 20.6^{#} | 390 ± 184.3^{#} | 6076.8+ 1091 | 4.3±1.2^{##} | 31.3±15.1^{##} |
| Cardiotonic Pill group | Before treatment | 108.8±28.3 | 233.1± 70.2 | 5863.3± 1072.7 | 5.9±2.0 | 18.5±7.6 |
| | After treatment | 158.2± 40.7^{##**} | 328.4± 78.5^{##*} | 5582.8± 1094.7^{#*} | 4.1± 1.6^{##**} | 36.4±6.7^{##**} |

| | | | | | | |
|---|---|---|---|---|---|---|
| Note: Comparing those before treatment with those after treatment, #p>0.05, ##p<0.05, *p>0.05, **p<0.05. | | | | | | |

The conclusion: Cardiotonic Pill has the function of antioxidation, and can lighten the lipid peroxidation reaction and raise the ability of antioxidation of human body.

### Treatment with Cardiotonic Pill for Essential Hypertension

The method:
(1) The choice of patients: Select those patients suffering from Phase I or II essential hypertension, but without secondary hypertension or cardiac, hepatic and renal insufficiencies.
(2) The administration: Stop the patients from taking any western and traditional Chinese medicines (except for hypotensors) for two weeks, and then, in the third week, measure their blood pressures and blood rheologyical indices and take down their clinical manifestations. The double blind method is adopted. In the Cardiotonic Pill group, the subjects take orally Cardiotonic Pill, 10 pills/time, and 3 times/day. In Compound Danshen Tablet group, the subjects take orally Compound Danshen Tablet, 5 tablets/time, and 3 times/day. The subjects in the control group take placebos, and the period of treatment is six weeks.

The results:
(1) Effect on the viscosity of whole blood. After treatment, the viscosities of whole blood of patients in both the Cardiotonic Pill group and the Compound Danshen Tablet group drop remarkably, but the curves of the viscosities of whole blood in the Cardiotonic Pill group at any shear rates go down more sharply than those in the Compound Danshen Tablet group. See **Table 15.**

**Table 15. Effect of Cardiotonic Pill on the Viscosity of Whole Blood (mPas, x±s)**

| Groups | | 3.83s⁻¹ | 28.3 s⁻¹ | 192 s⁻¹ |
|---|---|---|---|---|
| Cardiotonic group (30 subjects) | Before treatment | 18.27±2.85 | 5.82±0.93 | 4.54±0.78 |
| | After treatment | 11.79± 3,75^{**##&&} | 4.78± 0.84^{**##&&} | 3.98± 0.65^{**##&&} |
| Compound Danshen Tablet group (30 subjects) | Before treatment | 17.69±1.96 | 5.86±0.79 | 4.69±0.54 |
| | After treatment | 14.68± 3.41^{**&&} | 5.69±0.81^{*&} | 4.42±0.59^{**&&} |
| Control group (15 subjects) | Before treatment | 17.65 ±2.07 | 5.69 ±0.79 | 4.75±0.58 |
| | After treatment | 18.02±2.32 | 5.71±2.76 | 4.86±0.65 |

| | | | | |
|---|---|---|---|---|
| Note: In comparison with those of the same group before treatment, *p<0.05, **P<0.01. Comparing with those of the Cardiotonic Pill group after treatment, #p<0.05, ##p<0.01. Comparing with those of the control group after treatment, &p<0.05, &&p<0.01. | | | | |

(2) Effect on the deformation and the aggregation of erythrocytes. After treatment, the deformation of erythrocytes in Cardiotonic Pill group is markedly greater than that before treatment, and the area and the index of aggregation are observably smaller than those before treatment. And comparing with the Compound Danshen Tablet group, the Cardiotonic Pill group declines much faster (p<0.01). See **Table 16.**

**Table 16. Effect of Cardiotonic Pill on the Deformation and the Aggregation of Erythrocytes (x±s)**

| Groups | | Index of Deformation | Index of Aggregation | Area of Aggregation(integral) |
|---|---|---|---|---|
| Cardiotonic group (30 subjects) | Before treatment | 0.4115± 0.0360 | 4.06±0.39 | 841.12±67.68 |
| | After treatment | 0.4274± 0.034^{*&} | 3.41 ± 0.36^{**#&&} | 683.52±69.09^{**#&&} |
| Compound Danshen Tablet group (30 subjects) | Before treatment | 0.4066± 0.0290 | 3.98±0.34 | 806.90±66.30 |
| | After treatment | 0.4180± 0.0281^{*&} | 3.64± 0.39^{**&&} | 716.12±84.29^{**&} |
| Control group (15 subjects) | Before treatment | 0.4091± 0.0376 | 4.02±0.41 | 812.52±65.64 |
| | After treatment | 0.4001± 0.0381 | 4.01±0.39 | 804.44±68.06 |

| | | | | |
|---|---|---|---|---|
| Note: In comparison with those of the same group before treatment, *p<0.05, **P<0.01. Comparing with those of the Cardiotonic Pill group after treatment, #p<0.05, ##p<0.01. Comparing with those of the control group after treatment, &p<0.05, &&p<0.01. | | | | |

(3) Effect on Blood Pressure. After treatment, the systolic pressures and the diastolic pressures of patients in both the Cardiotonic Pill group and the Compound Danshen Tablet group drop markedly (p<0.01), and there is no evident difference between the two groups. See **Table 17.**
(4) Effect on symptoms of hypertension. The patients in both the Cardiotonic Pill group and the Compound Danshen Tablet group improve a lot in terms of such symptoms as headache, dizziness, and numb extremities (p<0.01), but do not improve in insomnia. See **Table 18.**

**Table 17. Effect of Cardiotonic Pill on Blood Pressure (mmHg, x±s)**

| Groups | | Systolic pressure | Diastolic pressure |
|---|---|---|---|
| Cardiotonic group (30 subjects) | Before treatment | 155.00±8.08 | 90.70±7.93 |
| | After treatment | 149.20±8.89**&& | 86.59±8.30**&& |
| Compound Danshen Tablet group (30 subjects) | Before treatment | 152.93±9.59 | 92.59±8.30 |
| | After treatment | 146.02±10.20**&& | 88.55±7.22**&& |
| Control group (15 subjects) | Before treatment | 154.06±7.05 | 90.90±9.10 |
| | After treatment | 152.08±9.25 | 91.10±8.70 |

| | | | |
|---|---|---|---|
| Note: In comparison with those of the same group before treatment, **P<0.01. Comparing with those of the control group after treatment, &&p<0.01. | | | |

**Table 18. Effect of Cardiotonic Pill on Clinical Symptoms of Hypertension (patients)**

| Groups | | Headache | | Dizziness | | Numb extremities | | Insomnia | |
|---|---|---|---|---|---|---|---|---|---|
| | | Yes | No | Yes | No | Yes | No | Yes | No |
| Cardiotonic group (30 subjects) | Before treatment | 20 | 10 | 16 | 14 | 12 | 18 | 8 | 22 |
| | After treatment | 7 | 23* | 2 | 28* | 3 | 27* | 4 | 26 |
| Compound Danshen Tablet group (30 subjects) | Before treatment | 22 | 8 | 17 | 13 | 10 | 20 | 6 | 24 |
| | After treatment | 15 | 15* | 8 | 22* | 3 | 27* | 6 | 248 |
| Control group (15 subjects) | Before treatment | 8 | 7 | 6 | 9 | 4 | 11 | 4 | 11 |
| | After treatment | 7 | 8 | 5 | 10 | 3 | 12 | 2 | 13 |

| | | | | | | | | | |
|---|---|---|---|---|---|---|---|---|---|
| Note: In comparison with those of the same group before treatment, *P<0.01. | | | | | | | | | |

The conclusion: The rheological property of erythrocytes of patients suffering from essential hypertension is clearly abnormal. The deformation of erythrocytes debases clearly, while the aggregation rises distinctly. The experiment shows that Cardiotonic Pill has the functions of markedly reducing the viscosity of whole blood and the index of and the area of aggregation of erythrocytes, and greatly raising the ability of deformation of erythrocytes. Cardiotonic Pill can also lower the blood pressure and improve clinical symptoms of the patients. And thus, Cardiotonic Pill is of great benefit to preventing or postponing the occurrence of essential hypertension and cardio-cerebral syndrome.

### Toxicity

DSP is safe and nontoxic. In China, over 5 million patients have been treated with DSP without severe side effects. Mild side effects, headache or dizziness, have been reported in a minor portion of patients.

### Summary of clinical studies

DSP is a new generation Chinese medicine for treating coronary heart disease. Clinical studies demonstrate that DSP alleviates as well as prevent angina by improving cardiac function, reducing myocardial ischemia, inhibiting platelet activation thus reducing blood stasis, and decreasing plasma cholesterol. The therapeutic efficacy of DSP was superior to another form of Chinese medicament Dan Shen tablets. DSP is as efficient as drugs used in the USA. DSP is aleviate angina as efficiently as nitroglycerine, prevents angina as efficiently as the long-acting nitrate isosorbid dinitrate, and inhibits platelet aggregation as effectively as aspirin. DSP is nontoxic: over 5 million people have been treated without noticeable side effects in most cases.

### PRECLINICAL STUDIES

Preclinical studies reveal the mechanism by which DSP prevent as well as alleviate angina.

### DSP alleviate angina by increasing blood flow.

Wistar rats, approximately 260 g, were anesthetized by urethane, opened the chest, excise the heart, perfused in the Langendorff mode at 37°C. And constant coronary perfusion pressure of 65 cm H₂O.

After stabilized the heart rate, various amounts of DSP or Danshen tablet were applied each time through lateral branch of aorta cannula. Subsequently, the coronary flow and the heart rate were measured. DSP increased coronary flow in a wide dosage. Danshen tablet, on the other hand, increased coronary flow at a narrow dosage. See **Table 19** below.

**Table 19. DSP increases coronary flow**

| | | Coronary flow | |
|---|---|---|---|
| | Dose (mg/ml) | Pretreatment | post-treatment |
| | None | 7.0 ± 1.1 | 7.1 ± 0.97 |
| DSP | 5.8 | 7.2 + 1.1 | 7.0 ± 1.4 |
| | 290 | 6.7 ± 1.6 | 8.7 ± 1.4 |
| | 580 | 6.7 ± 1.5 | 9.3 ± 2.9 |
| DS Tablet | 5.8 | 6.7 ± 1.4 | 7.1 ± 1.5 |
| | 290 | 7.3 ± 1.7 | 9.1 ± 2.1 |
| | 580 | 6.8 ± 1.4 | 7.1 ± 1.5 |

The effect of DSP and Danshen tablets on heart rate was examined. Neither DSP nor Danshen tablet changes heart rate. See **Table 20** below.

**Table 20. DSP does not increase heart rate**

| | | Heart rate | |
|---|---|---|---|
| | Dose (mg/ml) | Pretreatment | post-treatment |
| | None | 194 ± 17 | 193 + 12 |
| DSP | 5.8 | 180 ± 11 | 189 ± 9 |
| | 290 | 188 ± 7 | 184 ± 8 |
| | 580 | 173 ± 14 | 167 ± 13 5.8 |
| DS tablet | 5.8 | 180 ± 11 | 189 ± 9 |
| | 290 | 189 ± 16 | 183 ± 14 |
| | 580 | 186 ± 23 | 171 ± 8 |

### DSP increases coronary flow by relaxing vascular smooth muscle thus dilating vessels.

The effect of DSP on potassium-induced vasocontraction of rabbit aortic strip was examined. DSP relaxed smooth muscle thus dilating vessels significantly. Similar effects were observed in the experiments using pig coronary artery rings.

### DSP inhibits platelet aggregation

The effect of DSP on platelet aggregation was examined. Rabbit platelets were treated with DSP and aggregation was examined. DSP inhibited platelet aggregation significantly. See Table 21 below.

**Table 21. DSP inhibits platelet aggregation**

| DSP(mg/ml) | #animals | Aggregation ratio(%) | inhibition(%) |
|---|---|---|---|
| 0 | 6 | 6.7±4.4 | 0 |
| 1.8 | 6 | 42.7±2.5 | 8.3±4.5 |
| 3.5 | 6 | 33.4±3.4 | 23.6±6.7* |
| 7 | 6 | 25.3±2.1 | 37.6±5.9* |
| 14 | 6 | 15.8±3.0 | 69.0±6.9* |

Table 22 shows micronucleus rates in mice at Different Time After treatment with the herbal composition of this invention (8400 mg/kg).

**Table 22.**

| Time(h) | Polychromatic RBCs | Micronucleus Cells | Micro-rates (%) (x±SD) |
|---|---|---|---|
| 12 | 6,000 | 9 | 1.5±0.8 |
| 24 | 6,000 | 11 | 1.8±0.7 |
| 36 | 6,000 | 11 | 1.8±1.2 |
| 48 | 6,000 | 11 | 1.8±1.5 |
| 72 | 6,000 | 13 | 2.2±0.7 |
| Solvent 24 | 6,000 | 9 | 1.5±1.4 |

**Table 23** shows the micronuleus rates in Mice at 24 hrs after DSP & CP Administration.

**Table 23.**

| Dose(mg/kg) | Polychromatic RBCs | Micronucleus Cells | Micro-rates(%) (x±SD) |
|---|---|---|---|
| 8,400 | 6,000 | 12 | 2.0±0.6 |
| 840 | 6,000 | 9 | 1.5±1.0 |
| 84 | 6,000 | 11 | 1.8±1.0 |
| Solvent | 6,000 | 9 | 1.5±1.4 |
| CP (80mg/kg) | 6,000 | 138 | 23.0±4.0* |

| | | | |
|---|---|---|---|
| * Compare with solvent P<0.01. | | | |

**Table 24** shows assessment criteria for graded effectiveness of tested drugs.

**Table 24.**

| Parameters\ Effects | Very high effect | High Effect | Effect | No effect |
|---|---|---|---|---|
| RBC aggregation status | No | A little | Marked | Severe |
| onset Time | <90 | 90-180 | 180-300 | >300 |
| Micro-blood flow status | Better than normal | Turn to normal | Improved | Deteriorated |
| Duration (min) | >15 | 15-10 | 10-5 | <5 |

### DSP improves microcirculation

The effect of DSP on microcirculation in Chinese hamsters was examined. DSP improved microcirculation for 23 minutes within 111 minutes of buccal administration in all animals. See **Table 25** below.

**Table 25. DSP improves microcirculation**

| | onset Time(min) | Duration (min) | Effect rate (%) |
|---|---|---|---|
| DSP | 111 | 23 | 100 |

**Table 26** shows the protective effect of DSP on the myocardium induced by pituitrin in rats (second period).

**Table 26. (n=8)**

| Group | Dosage (g/kg) | Before medication | After medication | |
|---|---|---|---|---|
| | | | Before pituitrin | After pituitrin(40s-15 minutes) Number of rats with abnormal ECG |
| Control | | Normal | Normal | 7 |
| DSP | 0.4 | Normal | Normal | 3* |
| | 0.8 | Normal | Normal | 1** |
| | 1.2 | Normal | Normal | 1** |
| DST | 0.4 | Normal | Normal | 4 |
| | 0.8 | Normal | Normal | 1** |

| | | | | |
|---|---|---|---|---|
| Compared with the control. *p<0.05; **p<0.01. | | | | |

**Table 27** shows the inhibitory effect of DSP on myocardial ischemia induced by pituitrin in rat.

**Table 27.**

| Group | Dosage(g/kg) | Inhibition(%) |
|---|---|---|
| DSP | 0.4 | 71.4* |
| | 0.8 | 85.7* |
| | 1.2 | 71.4* |
| DST | 0.4 | 42.8* |
| | 0.8 | 85.7* |

| | | |
|---|---|---|
| As compared with the control. *p<0.05. | | |

**Table 28** shows the protective effect of DSP on the myocardial ischemia induced by pituitrin in rats (first period).

**Table 28 (n=8)**

| | | Changes in T-ST of ECG- (lead II) in the first period | | | | |
|---|---|---|---|---|---|---|
| Group | Dosage (g/kg) | | After medication | | | |
| | | Before medication | Before pituitrin | After pituitrin (0-40s.) Number of rats with abnormal ECG | | |
| | | | | elevated T | inverte d T | Total |
| Control | - | Normal | Normal | 4 | 3 | 7 |
| | 0.4 | Normal | Normal | 3 | 0 | 3* |
| DSP | 0.8 | Normal | Normal | 1 | 1 | 2* |
| | 1.2 | Normal | Normal | 2 | 1 | 3* |
| | 0.4 | Normal | Normal | 3 | 2 | 5 |
| DST | 0.8 | Normal | Normal | 1 | 1 | 2* |

| | | | | | | |
|---|---|---|---|---|---|---|
| As compared with control. *p<0.05. | | | | | | |

### Cardiotonic Pill's Action of Clearance on Oxygen Free Radicals

The study of Cardiotonic Pill's action of clearance on oxygen free radicals is carried out by using electron paramagnetic resonance (EPR) and spin trapping, with superoxide anions and hydroxy radicals produced by using the xanthine- xanthine oxidase system and the H₂O₂-Fe²⁺ system respectively.

The method:
(1) Production of hydroxy radicals. Set up a test model according to the Fenton Principle. Mix up H₂O₂, ferrisulphas and DMPO (5,5-dimethyl-pyrroline-1-oxide), and then carry out the EPR testing. The resulting signals serve as the control. Add Cardiotonic Pill in the treatment group.
(2) Production of superoxide anions. Set up a test model based on the xanthine- xanthine oxidase reaction. Mix up xanthine, Dietrylene triamine Pentacetic acid, DMPO and xanthine oxidase, and then carry out the EPR testing. The resulting signals serve as the control. Add Cardiotonic Pill in the treatment group.

Ten samples for each of the four groups are tested, and the results are expressed in terms of the average. T-test is applied in the statistical analysis.

The Results:
(1) Cardiotonic Pill's action of clearance on hydroxy radicals produced by the H2O2-Fe2+ system. An adduct DMPO-OH will be generated when a hydroxy radical is captured by a DMPO. The peak value of the adducts in the control group is 11.8±0.6 relative units , while that in the Cardiotonic Pill group is 4.1±0.5 relative units. There is a significant difference between them (p<0.01), and the clearance rate of Cardiotonic Pill is over 65%.
(2) Cardiotonic Pill's action of clearance on superoxide anions produced by the xanthine- xanthine oxidase system. An adduct DMPO-OOH will be generated when a superoxide anion is captured by a DMPO. The peak value of the adducts in the control group is 10.6±0.67 relative units ,while the spectral signals in the Cardiotonic Pill group disappear completely. In comparison with the control group, there is a significant difference (p<0.01), and the clearance rate of Cardiotonic Pill is 100%.

The above experiments show that Cardiotonic Pill has an effective action of clearance on superoxide anions produced by the xanthine- xanthine oxidase system and hydroxy radicals produced by the H2O2-Fe2+ system.

### Cardiotonic Pill's Effect on Free Radicals of Cerebral Ischemia Reinfusion Injury Tissue of Rats

The method: Take 30 SD rats, and randomly divide them into 3 groups, which are the feigned operation group (An operation is performed, but the blood vessels and nerves are not ligated), the cerebral ischemia reinfusion model group and the Cardiotonic Pill group (4g/kg). After the 3-day continuous intraperitoneal (Ip) administration, and two hours after the administration on the third day, the rats are anaesthetized with the 20% Ethylurethanm and their conducting arteries in both sides of their necks and vagus nerves are separated and ligated. After a 30-minute reinfusion, cut off their heads and get their brains. Take about 500mg of cortical tissues of their left-brains and hippocampus tissues in both sides, and put them into the liquid nitrogen for homogenization. After they are made into a homogenate with freezing physiological saline, centrifugate the homogenate. Take the supernatant and determine the activities of CAT and SOD and the contents of MAD and GSH.

The results: See **Table 29**.
(1) Cardiotonic Pill's effect on the activity of CAT and the content GSH of brain tissues. The activities of CAT of brain cortex and hippocampus tissues and the content of GSH of the brain cortex in the cerebral ischemia reinfusion model group are much lower than those in the feigned operation group. Both the activity of CAT of hippocampus tissues and the content of GSH of the brain cortex in the Cardiotonic Pill group are significantly greater than those in the model group.

**Table 29. The Activities of CAT and SOD and the Contents of MDA and GSH of Brain Tissues of the Rats (mPas, x±s) (n=10)**

| Groups | CAT Activity (U/mg Protein) | | GSH (µ mol/mg Protein) | | SOD Activity (U/mg Protein) | | MDA (nmol/mg Protein) | |
|---|---|---|---|---|---|---|---|---|
| | Brain Cortex | Hippocampus | Brain Cortex | Hippocampus | Brain Cortex | Hippocampus | Brain Cortex | Hippocampus |
| Feigned Operation Group | 2.94± 0.17 | 2.78±0.14 | 18.49 ±0.70 | 16.87±0.92 | 14.24 ±0.90 | 20.34±0.74 | 0.502 ± 0.054 | 1.084± 0.117 |
| Model Group | 2.17± 0.24⁸ | 1.97±0.23⁸ | 14.76 ± 1.12⁸ | 13.80±0.74 | 10.44 ± 0.79⁸ | 18.18± 0.62⁸ | 0.718 ± 0.070⁸ | 1.449± 0.140⁸ |
| Cardiotonic Pill Group | 2.55± 0.35 | 2.79±0.21³ | 17.14 ±0.76 | 15.76± 1.18³ | 13.31 ± 0.77³ | 20.59± 0.59³ | 0.483 ± 0.065³ | 1.069± 0.131³ |

| | | | | | | | | |
|---|---|---|---|---|---|---|---|---|
| Note: In comparison with the feigned operation group, *p<0.05; in comparison with the model group, #p<0.05 | | | | | | | | |

(2) Cardiotonic Pill's effect on the activity of SOD and the content MDA of brain tissues. The activity of SOD of brain tissues in the model group is significantly lower than that in the feigned operation group, while the content of MDA is significantly higher. The activities of SOD of brain cortex and hippocampus tissues in the Cardiotonic Pill group increase significantly, while the content of MDA decreases significantly.

The conclusion: After cerebral ischemia reinfusion, the content of MDA in the brain tissues increases, while the content of GSH decreases. The activities of CAT and SOD, two important enzymes for clearing oxygen free radicals in the tissues, decrease significantly, which shows that during the course of cerebral ischemia reinfusion, a great lot of oxygen free radicals occur due to the failure of the function of the free radical clearance system. This leads to the lipid peroxidation, and then leads to the brain injury. Cardiotonic Pill can decrease the contents of MDA in the brain cortex and hippocampus tissues of reinfused rats and increase the content of GSH and the activities of CAT and SOD greatly, which shows that Cardiotonic Pill has the functions of markedly restraining the reactions of oxygen free radicals, controlling the lipid peroxidation and protecting damaged brain cells caused by ischemia reinfusion.

**The Antioxidation of Cardiotonic Pill in Chronic Hepatic Injury** The method: The CCL4-high-fat-low-protein induced mild chronic hepatic injury model of the Wister rat is adopted. In the Cardiotonic Pill group, perfuse Cardiotonic Pill into the rats' stomachs at the dose of 4g/kg, while in the normal and the model groups, perfuse the same amount of physiological saline. The activity of SOD is determined by using the xanthine oxidase method, while the content of the MDA is determined by using the improved thiobarbituric acid method.

The results: See **Table 30**. Comparing the model group with the normal group, the activity of SOD degrades, while that of MDA increases. In the Cardiotonic Pill group, however, the activity of SOD increases, while that of MDA degrades, making the Cardiotonic Pill group go back to the normal.

**Table 30. The Activities of SOD and the Contents of MDA in Chronic Hepatic Injury**

| Groups | Rats | SOD (NU/mg.pr) | MDA (nM/mg.pr) |
|---|---|---|---|
| Normal group | 12 | 1.717±0.521 | 15.21±4.35 |
| Model group | 12 | 1.326±0.3218 | 19.39±4.62* |
| Cardiotonic Pill group | 11 | 1.710±0.415# | 15.16±4.29# |

| | | | |
|---|---|---|---|
| Note: In comparison with the normal group, *p<0.05. In comparison with the model group, #p<0.05 | | | |

The conclusion: MDA is a major degradation product of the lipid peroxidation. MDA can badly damage the structure of the cell membrane, and then hepatic cells. SOD is a scavenger of super-oxide anion free radicals, and it can restrain the lipid peroxidation caused by free radicals. Cardiotonic Pill can significantly increase the activity of SOD and decrease the content of MDA, which will degrade the level of the lipid peroxidation and lighten the hepatic injury.

### DSP is not mutagenic

It was examined whether DSP is mutagenic in the Ames assay. DSP was not mutagenic. See **Table 31** below.

**Table 31. The mutagenicity of DSP (Ames test)**

| | | colony number per dish | | | |
|---|---|---|---|---|---|
| | S9 | TA97 | Ta98 | TA100 | TA102 |
| DSP | - | | | | |
| 0.0 | - | 141±13 | 36±3 | 161±21 | 303±44 |
| 0.5 | - | 140±17 | 35±2 | 148±21 | 288±15 |
| 5 | - | 136±14 | 34±4 | 154±26 | 280±16 |
| 50 | - | 133±20 | 33±5 | 142±25 | 292±37 |
| 500 | - | 109±15 | 34±3 | 154±16 | 311±32 |
| 5000 | - | 67±8* | 30±4 | 149±27 | 298±32 |
| 0.0 | + | 141±13 | 44±6 | 176±19 | 296±39 |
| 0.5 | + | 148±13 | 42±9 | 161±27 | 296±37 |
| 5.0 | + | 152±11 | 44±7 | 161±16 | 292±38 |
| 50.0 | + | 140±18 | 37±6 | 166±9 | 307 ± 55 |
| 500.0 | + | 135±21 | 42±7 | 152±22 | 287±16 |
| 5000.0 | + | 119±17 | 38±7 | 162±17 | 363±57 |
| Dexon | | | | | |
| 50 | - | 2155±814 | 952±187 | 831±114 | 1510±211 |
| 2-AF | | | | | |
| 40 | - | 125±18 | 38±3* | 161±16 | |
| | + | 1404±644* | 1598 ± 124 | 1222 ± 309 | |
| DAN | | | | | |
| 100 | - | 364±50 | | | |
| | + | 943 ±102* | | | |

### Production of crude drugs

### 1. Dan shen

(1) Quality control
   Crude drug Dan Shen was sampled from production arias throughout China. Chemical analysis was conducted on those samples from different bases for their chief components. The results showed that the quality of Dan Shen from an aria named Shangluo was the best of all and it proved that the climate of Shangluo is most suitable for the growth of Dan Shen. The active ingredients of Dan Shen such as tanshinone and salvianic acid A. were approved the best in quantity.
(2) Topography
   Shangluo is geographically located at the East Longitude 108°34' 20'' ∼111°1'25'' and North Altitude 33°2'30"∼ 34°24'40" with an average sea level of 900 meters. The region is an area with low and median high mountains and is free of pollutions. The unpolluted clean air circumstance makes it ideal for the growth of drug plants.
(3) Climate
   It is warm and semi-humid in climate, typical for the mountainous areas of transitional zone from the subtropics to the temperate. Affected by the South-eastern monsoons, it has obvious divisions for seasons and a great amount of rain. The precipitation of rainfall of the year in this area is estimated at 733.9-899 mm. The sunshine period is around 1874.1-2185 hours a year, with an annual sun irridiance of 119.57-124.36 kilocalories/cm². The temperature varies from 18°C - 40.8 °C. A frost-free period lasts 198-218 days of the year.
(4) Soil
   80% of the soil in Shangluo is arenaceous, most of the arable land comprises of neutral and alkalescent soil with a pH value of 6.5-8. Within 0-20 cm of the tillage layer, the soil nutrients consist of the following: 1.36% organic matter; 0.085% nitrogen; 18 ppm fast-effective phosphor; 136 ppm fast-effective potassium; and 60 ppm alkaline-hydrolyzed nitrogen. Heavy metals and other toxic matters contained in the soil do not exceed the agricultural standards set by the country. The region is rich in plants and animals. Local farmers use organic fertilizers.
(5) Heavy Metals:
   The eight metals, including Lead, cadmium, mercury, arsenic, etc., the pesticide residues, air and water all meet the country environment standards.
(6) Standardization
   The planting and plowing of Dan Shen follow the standard of Good Agriculture Practice (GAP). Technological know-how relevant to the growing of Dan Shen is compiled into a booklets and distributed among the Dan Shen growers in the base. During the planting seasons, technicians are sent to the fields to give growers training on the spot and offer technical support, so as to standardize the planting of Dan Shen on a large plantation-like scale.
(7) The Shangluo production base had bred and cultivated 20 different breeds of Dan Shen. Different Dan Shens were observed and compared in their growth situations, yield, appearance and chemical components for three years. The best breeds in quality chosen for large-scale plantation.
(8) Tissue cultivation and clone technology are adopted in the Dan Shen cultivation to accelerate the procedure and shorten the circle of growth.
(9) After a 30-day period of test-tube planting, an enlarged reproduction procedure is taken. It lasts 40 days before the root period. The root period will takes another 10 days that is for the plants to generate and enrich their roots. The rate of rooting usually reaches 90% or above. The plant can then be transferred to the nursery, where advanced spray irrigation device and conditions are controlled by computer technology. The plants can be transplanted to the outside field after a month cultivation in the nursery.
(10) According to the experts, the cultivated Dan Shen from our product base are not only in high quality but also productive for their productive roots (50% more in root weight than those from other areas), and high chemical composition (70% higher in active drug ingredients than samples from other places).

### 2. Natural Borneol

(1) Growth Conditions
   The growth areas of natural Borneol are Xinhuang county of Hunan province, China, a region with mid-height hills at a sea level of 300-600m. 60% of the area is covered by forest. The land consists of yellow and red sand soil with pH 5-6. No air or water pollutions are found within the region.
(2) Biological Characteristics of the tree
   Extremely strong growth capability, it grows in brushwood field, 300-400 trees per mu *(equal to 666.7m²) ;* Parts above branch leaves are collected; net gain is 1000 kg per mu.
(3) Reproduction and transplant
   Use grafting and cuttage technology for reproduction. The Plant grows in the nursery garden during the first year and is transplanted to the filed in the spring of the second year. The filed need to be fertilized and scarified periodically.
(4) The plant may grow up to 30 cm in height and 80-100 cm for the largest diameter. It contains natural Camphor in different parts of the plant: leafs, branches, trunk and roots. Among all, the leaves contain the most of Camphor content.
(5) The Borneol-type Camphor from Xinghuang is one of the natural variation of Cirnamonium glandullferm (wall) Noes. The Camphors trees vary in contents of Camphor and Borneol, Some are low in Borneol, high in Camphor while others high in Borneol and low in Camphor. Through an assay with large amount of samples and many HPLC analyses, we finally choose a kind of Camphor trees that contains over 80% Borneol with less impurity.

### 3. Radix Notoginseng

### a. Seeds of the plant

(1) Determination of Radix Notoginseng
   With the PCR reaction for molecular mass marking, Radix Notoginseng can be stained observed, Radix Notoginseng has its characteristic DNA fingerprinting.
(2) Shape and properties
   The Radix Notoginseng seed has a round, circular body. For seeds of different growth period, Radix Notoginseng has 2-year or 3-year seeds. The 2-year seed is 0.45-0.55 cm in diameter and 95-103 grams in weight for its dried grain. The 3-year seeds is 0.54-0.65 cm in diameter and 98-109 grams in weight for its dried grain.
(3) Suitable Temperature for seed sprouting
   The appropriate temperature for seed sprouting is 10-30 °C, the ideal temperature is 15-20 °C.
(4) Water Content
   The amount of water contained should be 60-70%, if water content is below 20% for a long period of time, the seeds will lose vivacity.
(5) Dormancy
   The seeds have a tendency to go through dormancy for 45-60 days after collection.
(6) Life Span
   The seeds have a life span of 15 days in natural state after they are collected.
(7) Requirements for storage of Seed
   Seeds for storage should be collected from the plants that grow more than two years and the tree should be growing prosperously for parts above the earth and pest-free. It is recommended that seeds be collected from three-year plants.
(8) Management for the seed reservation field
   Seed reservation field should be better managed than the regular production fields, contaminated plants should be disposed of at all times, pests should not come in contact with the buds under any circumstances. During period of sprouting of buds and leafs, 3000 ppm of YunDa-120 and 400 times solution of Yang Kang biological fertilizer should be sprayed twice. And, during florescence and fruit period, Phytokinin is sprayed.
(9) Harvest Period of the seed
   The harvest period of Radix Notoginseng seed is from the end of October to the beginning of December.
(10) Methods for Collection of Seeds
   The collection of the seeds is decided dependant upon the levels of maturity of the seeds. The seeds from trees that grow stronger will be collected and stored separately. The base strictly prohibits collection of immature plants.
(11) Processing
   Plants are washed immediately after collection while pulps and blighted seeds are picked out. Dry the plants in the sun after washing.
(12) Storage
   Use 300 times solution of 58% metalaxyl manganous zinc dissolvable liquid to treat Radix Notoginseng seeds for 30 minutes, let the surface of the seeds become dry, and store the seeds with wet sand containing 20% water. It is a crucial step for the process.
(13) Packaging
   Final products are packaged in uncontaminated containers, There should be signs that indicate the date of collection, processing, and the product batch number.
(14) Transporting
   Clean, waterproof and ventilated transportation vehicles instruments should be used for transportation in order to prevent the product being contaminated with toxic matters. If it takes more than 8 hours for transportation, the product seed should be with the wet sand.
(15) Test for vitality of the seeds
   Use the TTC methods: weigh accurately 1 g of tetrazolium powder and dissolve it in 1000 mL of distilled water to make up the solution of 0.1% TTC. Immerse the sample into the solution and keep it for 24 hours, take it out and cut it into half and place one-half into a culture disk. Use the prepared 0.1% TTC to dye the sample for 30 minutes. The vitality of the seeds can be determined by the color of the seeds
(16) Inspection of Pests
   i. Observe the seed with human eyes while placing 500-1000 sample pellets on a white sheet of paper or glass. If unusual spots or pests appear on the surface of the sample, the contamination can be decided. Contaminated samples should be taken apart and be identified for its level of contamination.
   ii. Cutting and Inspect: use a scalpel to cut and open 2 sample sets each contain 100 seeds. Calculate the number of contaminated seeds to determine the level of contamination.
   iii. Smell to inspect samples: place them in hand and detect them by nose for any moldy odor. Or simply leave sample in a cup containing heated water (60-70 °C), and covered for 2-3 minutes, pour out the water and smell the seeds. The seeds should send forth a delicate fragrance, if not, it is probably contaminated.
(17) Color Inspection
   An uncontaminated seed should have light yellow and white color.
(18) Microscopic Inspection
   Pick out 5 test sample sets by random (each sample contains no less than 50 ceeds), place the samples in a culture disk for 24 hours. Observe them under a microscope to detect them for any pathogenic bacteria, if so, calculate the level of contamination.

### b. Seedling of the plant

(1) Temperature for sprouting
   The temperature for Radix Notoginseng's sprouting is 1-20 °C, the ideal temperature is 15°C.
(2) Water Content
   The water content of the soil used for seedling plant is 20-25%.
(3) Storage
   The development of Radix Notoginseng from resting bud to sprouting requires 90 days of dormancy period. 100 ppm of gibberellin can help shorten the Radix Notoginseng's dormancy period.
(4) Refining
   Radix Notoginseng's refinery period is from the beginning of December to the end of January. While refining, the roots should be handled with care. Plant immediately after refinery.
(5) Transportation
   Radix Notoginseng cannot be transported over long distance, otherwise it will be damaged. If unavoidable, Radix Notoginseng should be placed in a ventilated container with soil, without direct exposure to sunlight.
(6) Quality Inspection
   i. For weight of each unit, choose 300-500 seedling plant as samples, put each 100 seedling plants in a group, weigh them on the balance and calculate the unit weight.
   ii. For pesticide inspection: set up four groups of samples each contains 100 seedling plants. Place samples on a glass disk and observe the samples with human eyes or 5-10 times magnifying lens to check for their pesticide.
   iii. Set up four groups of sample each contains 100 plants, slice the samples for an observation under the microscope.

### c. Culture of the seedling

(1) Field condition
   Radix Notoginseng seedling plants are cultured in the best-conditioned areas. The base uses centered culture and large-scaled cultural method.
(2) Environment
   The area is totally free of pollutions. The air quality is above level 2 of GB 3059-96 standard.
(3) Water Resources
   The water resources consist of rainwater, underground water, and natural running water. Water quality in the area is monitored with the GB 5084-92 standard.
(4) Soil
   Radix Notoginseng cannot be planted in oozy soil, the amount of heavy metals in the soil chosen for Radix Notoginseng must be within relevant country standard.
(5) Ideal Soil
   For a good result expected, we choose acidic soil (pH 5.5-7.0) with a slope no more than 15°. A level no more than 1600 m above the sea should be with 8-12% of sunshine, and level more than 1600 m above sea level should be 10-20% of sunshine.
(6) Temperature
   During sprout period the atmospheric temperature should be at 20-25°C, and the earth temperature is at 10-15°C. During bearing period is the best atmospheric temperature that is 20-25°C, and the best soil temperature that is 15-20°C.
(7) Water Content
   Water content of soil should be at 25-30%.
(8) Soil Preparation
   Soil plotting should be repeated for 3 times before planting, and soil be exposed under sunlight, it helps to exterminate bacteria substance in the soil.
(9) Handle with Soil
   For prevention of root damage, 75-100g of lime is used each square meter before transplanting.
(10) Construction and management of Shade
   The shade is 1.8m above ground with a 2m deep trench below the ground. Sunlight penetration is best at 8-10% if in area of no more than 1600 m above sea level, or 10-15% if it is more than 1600 m. The ground should be flat; deep layer of the earth should be lose; while the surface level of the earth should be rigid. Planting season is best at the end of December to the end of January. Before planting, the seeds should be immersed in 58% metalaxylic zinc (500-800x) or 1.5% antimycin (200ppm) for 30-50 minutes and dip out to let dry. This is to protect the plant from diseases (Coated seeds do not have to undergo the above procedures). Density of planting is to be 4 x 5 cm or 5 x 5 cm, with 100-200 thousand seeds per mu of land. Use special tools to create a shallow gutter and use machine or hands to seed and plant. Seeds are covered with fine soil completely.
   After all, fertilizing, watering and weeding tasks are performed. Weeds should be eliminated all the time. If the shade is broken, it should be repaired immediately and ensured the correct penetration of sunlight. Natural fertilizers including poultry waste, stove dust, and bone dust (human waste should not apply).

### d. Radix Notoginseng Cultivation

(1) Topology
   The ground is best be with a moderate slope under 15° and a good exposure to sunshine.
(2) Soil Texture
   It is the best if the ground is deeply seated with loose and sand soil.
(3) Soil pH should be at 5.5-7.
(4) Pre-planted Crops
   Corns, wheat, and beans are pre-planted in new fields in avoid of soil destruction.
(5) Sea Level
   1400-1800m above sea level near an altitude of 23.5° is the location of the most suitable Radix Notoginseng area for cultivation.
(6) Sunshine
   Radix Notoginseng is a kind of plant that requires only 8-20% of sunshine. The amount of sunshine should be varied increased in respect to different period of its growth. However, too much sunshine exposure will result in stagnant plants.
(7) Water Content
   The water content within the soil should be at 25-30%.
(8) Fertilizer
   Organic fertilizers are used along with compound fertilizers, micronutrient fertilizers, or trace-element fertilizers.
(9) Temperature
   The average temperature is estimated at 15-18°C during the year in the Radix Notoginseng area. During sprout period, atmospheric temperature should be, the most suitable, at 20-25°C, and soil temperature 15 °C. During nutrient development and blooming period, temperature is better be kept at 25°C. If the temperature is below 15 °C, florescence will be affected.
(10) Filed Division
   Before cultivation, the field should be ploughed and loosened 3 times until its structure becomes powderized.
(11) Soil Management
   Before sowing and transplanting, apply 75-100g of quick lime to soil for sterilization purposes.
(12) Standards of the bed
   Flat ground soil bed of 20-25 cm in height, at slope area it should be 15-20 cm. The width of the bed is between 120 and 140 cm in a shape of a tile. The soil at the bottom of the base should be loose and that on the top should be solid, that is for better penetration.
(13) Seed Soaking
   During transplanting, soak seed for 30-50 minutes in 58% metalaxyl (500-800x) and then let dry, this will prevent plant from diseases and eliminate pests.
(14) Density of planting
   Keep a distance of 10 x 12.5 cm - 10 x 15 cm in plant density. That is 26-32 thousand plants every mu.
(15) Methods for Transplanting
   Seedlings are planted facing the same direction for management purposes. In case of slope grounds, seedlings are planted from low end to the high ground. The first row of seedlings is facing up while the second row faces down. Buds are also to face upward, and the bottom is to face inward.
(16) Covering of Soil
   Use powdered, loose, and moistures soil to cover the seedlings completely, without exposing the roots or the buds.
(17) Fertilization
   Use poultry waste, stove ash, bone ash, Calcium Magnesium Phosphate, etc as specialized fertilizer.

### REFERENCES

1. US Pat. No. 5288485 "vasodilating agent"
2. US Pat. No. 5433957 "vasodilating agent"
3. US Pat. No. 5776463 "Method of reducing stress and circulatory heart disease with freeze-dried borage petal extracts"
4. Yang GY et al., "Clinical studies on the treatment of coronary heart disease with Valeriana officinalis varlatifolia" Chung Kuo Chung Hsi I Chieh Ho Tsa Chih, 14(9):540-2, 1994
5. Wu Y, "Clinical study on Xintongkang capsule in treating angina pectoris of coronary heart disease" Chung Hsi I Chieh Ho Tsa Chih, 10(7):395-8, 1990.
6. Liu KY et al., "Clinical observation on treatment of 45 angina pectoris patients of coronary heart disease with taponin" Chung Hsi I Chieh Ho Tsa Chih, 15 (11) :649-51, 1995.
7. Hu JX et al., "Clinical and experimental study of shenshao tongguan pian in treating angina pectoris of coronary heart disease" Chung Hsi I Chieh Ho Tsa Chih, 10(10) :596-9, 1990.
8. Li Y et al., "Effects of Kuo-guan granule on plasma zinc, copper and erythrocyte GSH-Px (glutathione peroxidase) in patients with angina pectoris" Chung Hsi I Chieh Ho Tsa Chih, 10(6):348-50, 1990.
9. Wang B et al., "406 cases of angina pectoris in coronary heart disease treated with saponin of Tribulus terrestris" Chung Hsi I Chieh Ho Tsa Chih, 10(2):85-7, 1990.
10. Jin C et al., "Effect of Astragalus membranaceus on erythrocyte sodium content and sodium transport in the coronary heart disease" Chung Hsi I Chieh Ho Tsa Chih, 11(11):651-3, 1991.
10. Jiang HW et al., "Clinical study in treating qi-deficiency and blood-stasis syndrome of angina pectoris with qi xue granule" Chung Kuo Chung Hsi I Chieh Ho Tsa Chih, 12(11):663-5, 1992
11. Wang XF et al., "Clinical observation of wenxin decoction in treating 82 patients with spontaneous angina pectoris" Chung Kuo Chung Hsi I Chieh Ho Tsa Chih, 16(4) :201-3, 1996.
12. Lei ZY et al., "Action of Astragalus membranaceus on left ventricular function of angina pectoris" Chung Kuo Chung Hsi I Chieh Ho Tsa Chih, 14(4):199-202, 1994.
13. He W et al., "Effects of Kuo guan powder on immunologic functions in patients with ben-xu biao-shi syndrome in ischemic heart disease" Chung Hsi I Chieh Ho Tsa Chih, 9(4):213-5, 1989.
14. Chen K et al., "Clinical study on the effect of shuxuening tablet in treatment of coronary heart disease" Chung Kuo Chung Hsi I Chieh Ho Tsa Chih, 16(1):24-6, 1996.
15. Zhang H et al., "Clinical study on effects of buyang huanwu decoction on coronary heart disease" Chung Kuo Chung Hsi I Chieh Ho Tsa Chih, 15(4):213-5, 1995.
16. Zhang XL et al., "Preliminary study of rose shu-xin oral liquid in the treatment of angina pectoris in coronary heart disease" Chung Kuo Chung Hsi I Chieh Ho Tsa Chih, 12 (7) :414-6, 1992.
17. Hu JX et al., "Clinical and experimental study of shenshao tongguan pian in treating angina pectoris of coronary heart disease" Chung Hsi I Chieh Ho Tsa Chih
18. Zhu W et al., "Protective effect of danshen during myocardial ischemia and reperfusion: an isolated rat heart study" American Journal of Clinical Medicine, 18(1-2):19-24, 1990.
19. Li YY, "Clinical and experimental study on the effect of xue mai tong on coronary heart disease" Chung Hsi I Chieh Ho Tsa Chih, 9(2):79-81, 1989.
20. Cai PY et al., "A clinical study of hehuantang in treating coronary heart disease" Chung Kuo Chung Hsi I Chieh Ho Tsa Chih, 16(4):204-6, 1996.
21. 22. Lin QC, "A clinical study of guan mai le in the treatment of coronary heart disease" Chung Hsi I Chieh Ho Tsa Chih, 9(5):280-2, 1989.
22. Li SQ et al., "Clinical observation on the treatment of ischemic heart disease with Astragalus menbranaceus" Chung Kuo Chung Hsi I Chieh Ho Tsa Chih, 15(2):77-80, 1995.
23. Lu BJ et al., "Effect of sheng mai san on lipid peroxidation in acute myocardial infarction patients" Chung Kuo Chung Hsi I Chieh Ho Tsa Chih, 14(12):712-4, 1994
24. Wang S et al., "Effects of Codonopsis pilosulae on the synthesis of thromboxane A2 and prostacyclin" Chung Hsi I Chieh Ho Tsa Chih, 10(7):391-4, 1990.
25. Guan M et al., "Observation on the treatment of coronary heart disease by kuo guan qu yu ling" Journal of Traditional Chinese Medicine 10(1):49-53, 1990.
26. "Fufang Danshen Pian "Pharmacopeia of the People's Republic of China. English Edition 1997. Volume 1, 280.
27. Zhu HG, "Clinical study on xinkening in treating asymptomatic myocardial ischemia in coronary heart disease" Chung Kuo Chung Hsi I Chieh Ho Tsa Chih, 14(4):213-5, 1994.
28. Chiang WT et al., "Effects of "Danshensu" and other two water-soluble components of salvia miltiorrhiza on dog ischemic myocardium and isolated pig coronary artery" Acta Acad Med Prim Shanghai 1982; 9: 13-19.
29. Sun Xi-ming et al., "Studies on a new pharmacological action of anextract of Danshen (Salvia miltiorrhiza) "Chin Med Herb 1991; 22:20-23.
30. Chen ZH et al., "Studies on effects of "Danshensu" on experimental microcirculatory disturbances and plasm lactic acid concentrations" Acta Acad Med Prim Shanghai 1987; 14: 25-29.
31. Huang C et al., "Effects of Panax Notoginseng Saponins on myocardial ischemia and reperfusion injury in conscious rabbit" Chin Bull Pharmacol 1991; 7: 190-3.
32. Shi L et al., "Effects of total saponins of Panax Notoginseng on increasing PGI2 in carotid artery and decreasing TXA2 in blood platelets" Acta Pharmcol Sin 1990; 11: 29-32.
33. Xu W et al., "Effect of menthol and borneol on the distribution of sulfadiazine sodium and Evan's blue in the rat and mouse brain" Pharmacol Chin Med Clin 1995; (6): 31-33.
34. Chen TF et al., "Enhancement of absorption of tetramethylpyrazine by synthetic borneol" Acta Pharmacol Sin 1990; 11:42-44.
35. Li X et al., "Protective effects of Panax notoginseng saponins on experimental myocardial injury induced by ischemia and reperfusion in rat" Acta Pharmacol Sin 1990;11:26-29.
36. Pan JG et al., "Chemical Studies on Essential oils from six Artemisia Species" Chin J Chin Mater Med 1992; 17: 741-6.
37. Wu YZ et al., "An assessment of Radix Silviae Miltiorrhizae in promoting blood circulation by removing blood stasis" Acta Nanjing Univ Trad Chin Mater Med 1995; 11:35-6.
38. Wang NS et al., "Experimental studies of "Benifits of Borneol as an Assistant or a guide, " J Trad Chin Med 1994; 35:46-7.
39. Li CZ et al., "Anti-coagulation effect of Radix Silviae Miltiorrhizae" Chin J Integr Med 1983; 3: 297-9.
40. Li CZ et al., "An experimental study on thrombotic inhibition effect of Radix Silviae Miltiorrhizae" Acta Acad Med Prim Shanghai 1979;6:145-9.
41. Mo QX et al., "Dynamics of 3H-Borneol" Propriet Trad Chin Med Res 1982; 8 : 5-7.
42. Zheng RX et al., "Preservation effect of Radix Silviae Miltiorrhizae on myocardial ischemia induced by coronary ligation in mice" Chin J Integr Med 1992;12:424-6.

## Claims

1. A process for preparing a Dan Shen pill (DSP) for use in treating chronic stable angina pectoris comprising the steps of:
a) extracting *Radix Salviae Miltorrhizae* and Panax Notoginsen separately in hot water reflux and filtering separately;
b) concentrating filtrates separately;
c) refining the concentrates separately using resin columns and concentrating, and getting refined water-soluble extract of *Radix Salviae Miltorrhizae* and refined water-soluble extract of Panax Notoginsen
d) mixing the refined water-soluble extracts from step (c) with synthetic borneol and pharmaceutical carriers.

2. The process of claim 1, wherein
(1) Extracting water-soluble components of Panax Notoginseng comprises the following steps:
(a) Dilution of herbs with 5-7 times of water;
(b) Extraction of water-soluble components of Panax Notoginseng by boiling in a tank with the air pressure between 0.04-0.06 mPa for 2 hours;
(c) Repeat extraction under the same condition for 1.5 hours;
(d) Filtration of the extraction with 100-mesh net;
(e) Refine the filtrate using macroporous adsorption resin eluting with ethanol;
(f) Concentration of the eluted extracts under decompressed condition with the air input to 0.04-0.06 mPa and the vacuum to -0.076∼0.088 mPa until the density is 1.33-1.35;
(2) Extracting water-soluble components of Radix Salviae Miltorrhizae comprises the following steps:
(a) Dilution of herbs with 5-7 times of water;
(b) Extraction of water-soluble components of Radix Salviae Miltorrhizae by boiling in a tank with the air pressure between 0.04-0.06 mPa for 2 hours;
(c) Repeat extraction under the same condition for 1.5 hours;
(d) Filtration of the extraction with 100-mesh net;
(e) Concentration of the filtrates under decompressed conditions with the vacuum pressure -0.076-0.088 mPa until one Kg initial herb becomes one liter;
(f) Precipitation of the concentrates with ethanol;
(g) Filtration of the ethanol precipitates solution through 100-mesh net;
(h) Concentration of the filtrates under decompressed conditions with input air pressure 0.04-0.06 mPa and the vacuum pressure 0.076-0.088 mPa;
(i) Refine the concentrates by polyamide chromatography eluting with ethanol;
(j) Concentrate the refined extracts to the density of 1.33-1.35;
(3) and further comprising producing the pill consisting in the following steps:
(a) Mixing the extracts of Panax Notoginseng, the extracts of Radix Salviae Miltorrhizae, synthetic boneol and polyethylene glycol 6000 at the ratio of 4.0:20.6:1.9:79.5;
(b) Melting the mixture;
(c) Manufacturing the melted mixture to pills using the dropping machine with the following characteristics: the temperature of dropping pot is constantly 89-93°C, the cooling solution is liquid paraffin of which the temperature is lower than 8°C, the inner diameter of the dropping head is 1.8mm, the outer diameter of the dropping head is 2.4mm, the distance between the dropping head and the surface of cooling solution is 15 cm.
(d) Centrifugation of the pills at 800-1100 rpm for 15 minutes to remove oils.

3. A Dan Shen pill for use in treating chronic stable angina pectoris obtainable by the process according to any one of claims 1-2.

4. The Dan Shen pill according to claim 3, comprising 5-40% water soluble phenolic acid of Radix Salviae Miltorrhizae, 1-10% water soluble saponin of Panax Notoginseng and 1-5% of Borneol.

5. The Dan Shen pill according to claim 3, comprising 10-30% water soluble phenolic acid of Radix Salviae Miltorrhizae, 2-6% water soluble saponin of Panax Notoginseng and 1-3% of Borneol.

6. The Dan Shen pill according to claim 3, comprising about 0.14 to about 0.18 mg Danshensu per pill, more than 12.12µg sanchinoside R1 per pill and more than 56.26µg ginsenoside Rg1 per pill.

## Patentansprüche

1. Verfahren zur Herstellung von Dan Shen-Pillen (DSP) zur Verwendung bei der Behandlung von chronischer stabiler Angina pectoris, das folgende Schritte umfasst:
a) Separates Extrahieren von *Radix Salviae Miltorrhizae* und *Panax Notoginseng* in heißes Wasser unter Rückfluss und separates Filtrieren;
b) Separates Konzentrieren der Filtrate;
c) Separates Reinigen der Konzentrate unter Verwendung von Austauschersäulen und Konzentrieren, um einen gereinigten, wasserlöslichen Extrakt von *Radix Salviae Miltorrhizae* und einen gereinigten, wasserlöslichen Extrakt von *Panax Notoginseng* zu erhalten;
d) Mischen der gereinigten, wasserlöslichen Extrakte vom Schritt c) mit synthetischem Borneol und pharmazeutischen Trägerstoffen.

2. Verfahren nach Anspruch 1, wobei
(1) das Extrahieren der wasserlöslichen Bestandteile von *Panax Notoginseng* die folgenden Schritte umfasst:
a) Verdünnen der Kräuter mit dem 5-7 Fachen an Wasser;
b) Extrahieren der wasserlöslichen Bestandteile von *Panax Notoginseng* durch Sieden in einem Behälter bei einem Luftdruck zwischen 0,04-0,06 mPa während 2 Stunden;
c) Wiederholen der Extraktion unter gleichen Bedingungen während 1,5 Stunden;
d) Filtrieren des Extrakts mit einem 100 mesh-Sieb;
e) Reinigen des Filtrats unter Verwendung von makroporösem Adsorptionsharz, wobei mit Ethanol eluiert wird;
f) Konzentrieren der eluierten Extrakte unter vermindertem Druck, bei einem Eingangsluftdruck von 0,04-0,06 mPa und einem Vakuum von 0,076∼-0,088 mPa, bis die Dichte 1,33 bis 1,35 beträgt;
(2) das Extrahieren der wasserlöslichen Bestandteile von *Radix Salviae Miltorrhizae* die folgenden Schritte umfasst:
a) Verdünnen der Kräuter mit dem 5-7 Fachen an Wasser;
b) Extrahieren der wasserlöslichen Bestandteile von *Radix Salviae Miltorrhizae* durch Sieden in einem Behälter bei einem Luftdruck zwischen 0,04-0,06 mPa während 2 Stunden;
c) Wiederholen der Extraktion unter gleichen Bedingungen während 1,5 Stunden;
d) Filtrieren des Extrakts mit einem 100 mesh-Sieb;
e) Konzentrieren der Filtrate unter vermindertem Druck, in einem Vakuum von 0,076-0,088 mPa, bis ein Kilogramm der anfänglichen Kräuter einen Liter ergibt;
f) Fällen der Konzentrate mit Ethanol;
g) Filtrieren der Ethanolpräzipitat-Lösung durch ein 100 mesh-Sieb;
h) Konzentrieren der Filtrate unter vermindertem Druck, bei einem Eingangsluftdruck von 0,04-0,06 mPa und einem Vakuum von 0,076- 0,088 mPa;
i) Reinigen der Konzentrate durch Polyamid-Chromatografie, wobei mit Ethanol eluiert wird;
j) Konzentrieren der gereinigten Extrakte zu einer Dichte von 1,33-1,35;
(3) und das Verfahren des Weiteren die Fertigung von Pillen umfasst, die aus folgenden Schritten besteht:
a) Mischen der Extrakte von *Panax Notoginseng* und *Radix Salviae Miltorrhizae* mit synthetischem Borneol und Polyethylengykol 6000 im Verhältnis von 4,0:20,6:1,9:79,5;
b) Schmelzen des Gemischs;
c) Verarbeiten des geschmolzenen Gemischs zu Pillen unter Verwendung einer Tropfen formenden Maschine mit den folgenden Merkmalen: Die Temperatur des Kessels beträgt konstant 89 bis 93 °C, als Kühllösung dient flüssiges Paraffin mit einer Temperatur von unter 8 °C, der Innendurchmesser des Tropfen formenden Kopfes beträgt 1,8 mm, der Außendurchmesser des Tropfen formenden Kopfes beträgt 2,4 mm, der Abstand zwischen dem Tropfen formenden Kopf und der Oberfläche der Kühllösung beträgt 15 cm;
d) Zentrifugieren der Pillen bei 800 bis 1100 Umdrehungen pro Minute für 15 Minuten, um Öle zu entfernen.

3. Dan Shen-Pillen zur Verwendung bei der Behandlung von chronischer stabiler Angina pectoris, die durch das Verfahren nach einem der Ansprüche 1 bis 2 erhalten werden können.

4. Dan Shen-Pillen nach Anspruch 3, die 5 bis 40 % wasserlösliche Phenolsäure von *Radix Salviae Miltorrhizae,* 1 bis 10 % wasserlösliches Saponin von *Panax Notoginseng* und 1 bis 5 % Borneol enthalten.

5. Dan Shen-Pillen nach Anspruch 3, die 10 bis 30 % wasserlösliche Phenolsäure von *Radix Salviae Miltorrhizae,* 2 bis 6 % wasserlösliches Saponin von *Panax Notoginseng* und 1 bis 3 % Borneol enthalten.

6. Dan Shen-Pillen nach Anspruch 3, die etwa 0,14 bis etwa 0,18 mg Danshensu pro Pille, mehr als 12,12 µg Sanchinosid R1 und mehr als 56,26 µg Ginsenosid Rg1 pro Pille enthalten.

## Revendications

1. Procédé de préparation d'une pilule de Dan Shen (DSP) pour son utilisation dans le traitement de l'angine de poitrine chronique stable comprenant les étapes suivantes :
a) l'extraction de *Radix Salviae Miltorrhizae* et de *Panax Notoginseng* séparément dans de l'eau brûlante à reflux et une filtration séparément ;
b) la concentration des filtrats séparément ;
c) le raffinage des concentrés séparément en utilisant des colonnes de résine et la concentration, et l'obtention d'un extrait hydrosoluble raffiné de *Radix Salviae Miltorrhizae* et d'un extrait hydrosoluble raffiné de *Panax Notoginseng ;*
d) le mélange des extraits hydrosolubles raffinés issus de l'étape (c) avec du bornéol synthétique et des supports pharmaceutiques.

2. Procédé selon la revendication 1, dans lequel
(1) l'extraction des composants hydrosolubles de *Panax Notoginseng* comprend les étapes suivantes :
(a) la dilution des herbes avec 5 à 7 fois d'eau ;
(b) l'extraction des composants hydrosolubles de *Panax Notoginseng* par ébullition dans une cuve avec la pression d'air entre 0,04 et 0,06 mPa pendant 2 heures ;
(c) la répétition de l'extraction dans les mêmes conditions pendant 1,5 heure ;
(d) la filtration de l'extraction avec un filet de 100 mesh ;
(e) le raffinage du filtrat en utilisant une résine d'adsorption macroporeuse s'éluant avec de l'éthanol ;
(f) la concentration des extraits élués dans des conditions de décompression avec l'entrée d'air à 0,04 à 0,06 mPa et le vide à -0,076 à 0,088 mPa jusqu'à une densité de 1,33 à 1,35 ;
(2) l'extraction des composants hydrosolubles de *Radix Salviae Miltorrhizae* comprend les étapes suivantes :
(a) la dilution des herbes avec 5 à 7 fois d'eau ;
(b) l'extraction des composants hydrosolubles de *Radix Salviae Miltorrhizae* par ébullition dans une cuve avec la pression d'air entre 0,04 et 0,06 mPa pendant 2 heures ;
(c) la répétition de l'extraction dans les mêmes conditions pendant 1,5 heure ;
(d) la filtration de l'extraction avec un filet de 100 mesh ;
(e) la concentration des filtrats dans des conditions de décompression avec une pression de vide de -0,076 à 0,088 mPa jusqu'à ce qu'un kg d'herbes initial devienne un litre ;
(f) la précipitation des concentrés avec de l'éthanol ;
(g) la filtration de la solution des précipités à l'éthanol à travers un filet de 100 mesh ;
(h) la concentration des filtrats dans des conditions de décompression avec une pression d'entrée d'air de 0,04 à 0,06 mPa et une pression de vide de 0,076 à 0,088 mPa ;
(i) le raffinage des concentrés par chromatographie sur polyamide s'éluant avec de l'éthanol ;
(j) la concentration des extraits raffinés jusqu'à la densité de 1,33 à 1,35 ;
(3) et comprenant en outre la production de la pilule consistant en les étapes suivantes :
(a) le mélange des extraits de *Panax Notoginseng,* des extraits de *Radix Salviae Miltorrhizae,* du bornéol synthétique et de polyéthylène glycol 6000 dans un rapport de 4,0/20,6/1,9/79,5 ;
(b) la fusion du mélange ;
(c) la transformation du mélange fondu en pilules en utilisant la machine de déversement avec les caractéristiques suivantes : la température de la cuve de déversement est constamment de 89 à 93 °C, la solution de refroidissement est de la paraffine liquide dont la température est inférieure à 8 °C, le diamètre interne de la tête de déversement est de 1,8 mm, le diamètre externe de la tête de déversement est de 2,4 mm, la distance entre la tête de déversement et la surface de la solution de refroidissement est de 15 cm ;
(d) la centrifugation des pilules à 800 à 1100 tr/min pendant 15 minutes pour éliminer les huiles.

3. Pilule de Dan Shen pour une utilisation dans le traitement d'une angine de poitrine chronique stable pouvant être obtenue par le procédé selon l'une quelconque des revendications 1 et 2.

4. Pilule de Dan Shen selon la revendication 3, comprenant 5 à 40 % d'acide phénolique hydrosoluble de *Radix Salviae Miltorrhizae,* 1 à 10% de saponine hydrosoluble de *Panax Notoginseng* et 1 à 5 % de bornéol.

5. Pilule de Dan Shen selon la revendication 3, comprenant 10 à 30 % d'acide phénolique hydrosoluble de *Radix Salviae Miltorrhizae,* 2 à 6% de saponine hydrosoluble de *Panax Notoginseng* et 1 à 3 % de bornéol.

6. Pilule de Dan Shen selon la revendication 3, comprenant environ 0,14 à environ 0,18 mg de Danshensu par pilule, plus de 12,12 µg de sanchinoside R1 par pilule et plus de 56,26 µg de ginsénoside Rg1 par pilule.
